(19) Europäisches Patentamt
European Patent Office
Office européen des brevets

(11) EP 2 377 860 A1

(12) **EUROPEAN PATENT APPLICATION**

(43) Date of publication:
**19.10.2011 Bulletin 2011/42**

(21) Application number: **11162677.6**

(22) Date of filing: **15.04.2011**

(51) Int Cl.:
***C07D 401/04*** *(2006.01)* ***C07D 401/12*** *(2006.01)*
***C07D 401/14*** *(2006.01)* ***C07D 413/14*** *(2006.01)*
***C07D 471/04*** *(2006.01)* ***A61K 31/496*** *(2006.01)*
***A61P 25/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **16.04.2010 EP 10160190**

(71) Applicant: **AC Immune S.A.**
**1015 Lausanne (CH)**

(72) Inventors:
- **Kroth, Heiko**
  **1024 Ecublens (CH)**
- **Hamel, Cotinica**
  **1030 Bussigny-près-Lausanne (CH)**
- **Benderitter, Pascal**
  **1350 Orbe (CH)**
- **Froestl, Wolfgang**
  **1024 Ecublens (CH)**
- **Sreenivasachary, Nampally**
  **1024 Ecublens (CH)**
- **Muhs, Andreas**
  **1009 Pully (CH)**

(74) Representative: **Vossius & Partner**
**Siebertstrasse 4**
**81675 München (DE)**

(54) **Novel compounds for the treatment of diseases associated with amyloid or amyloid-like proteins**

(57) The present invention relates to novel compounds that can be employed in the treatment of a group of disorders and abnormalities associated with amyloid protein, such as Alzheimer's disease, and of diseases or conditions associated with amyloid-like proteins. The compounds of the present invention can also be used in the treatment of ocular diseases associated with pathological abnormalities/changes in the tissues of the visual system. The present invention further relates to pharmaceutical compositions comprising these compounds and to the use of these compounds for the preparation of medicaments for treating or preventing diseases or conditions associated with amyloid and/or amyloid-like proteins. A method of treating or preventing diseases or conditions associated with amyloid and/or amyloid-like proteins is also disclosed.



Printed by Jouve, 75001 PARIS (FR)

Sequence Listing

<110> AC Immune S.A.

<120> Novel compounds for the treatment of diseases associated with amyloid or amyloid-like proteins

<130> R2203 EP

<160> 4

<170> PatentIn version 3.3

<210> 1
<211> 112
<212> PRT
<213> Artificial sequence

<220>
<221> source
<223> /note="Description of artificial sequence: artificial humanized C2 HuVK 1 variable light chain"

<400> 1

```
Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Val Tyr Ser
            20                  25                  30

Asn Gly Asp Thr Tyr Leu His Trp Tyr Leu Gln Lys Pro Gly Gln Ser
        35                  40                  45

Pro Gln Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Ser
                85                  90                  95

Thr His Val Pro Trp Thr Phe Gly Gln Gly Thr Lys Val Glu Ile Lys
            100                 105                 110
```

<210> 2
<211> 219
<212> PRT
<213> Artificial sequence

<220>
<221> source
<223> /note="Description of artificial sequence: artificial humanized C2 light chain"

<400> 2

```
Asp Ile Val Met Thr Gln Ser Pro Leu Ser Leu Pro Val Thr Pro Gly
1               5                   10                  15
```

```
Glu Pro Ala Ser Ile Ser Cys Arg Ser Ser Gln Ser Leu Val Tyr Ser
            20                  25                  30

Asn Gly Asp Thr Tyr Leu His Trp Tyr Leu Gln Lys Pro Gly Gln Ser
        35                  40                  45

Pro Gln Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Ser
                85                  90                  95

Thr His Val Pro Trp Thr Phe Gly Gln Gly Thr Lys Val Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215
```

<210> 3
<211> 112
<212> PRT
<213> Artificial sequence

<220>
<221> source
<223> /note="Description of artificial sequence: artificial humanized C2 HuVH AF 4 variable heavy chain"

<400> 15

```
Glu Val Gln Leu Val Glu Ser Gly Gly Gly Leu Val Gln Pro Gly Gly
1               5                   10                  15
```

```
Ser Leu Arg Leu Ser Cys Ala Ala Ser Gly Phe Thr Phe Ser Ser Tyr
            20                  25                  30

Gly Met Ser Trp Val Arg Gln Ala Pro Gly Lys Gly Leu Glu Leu Val
        35                  40                  45

Ala Ser Ile Asn Ser Asn Gly Gly Ser Thr Tyr Tyr Pro Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Ser Gly Asp Tyr Trp Gly Gln Gly Thr Thr Val Thr Val Ser Ser
            100                 105                 110

<210>  4
<211>  439
<212>  PRT
<213>  Artificial sequence

<220>
<221> source
<223> /note="Description of artificial sequence: artificial humanized C2 heavy
chain"

<400>  4

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Gly Met Ser Trp Val Arg Gln Ala Pro Gly Lys Gly Leu Glu Leu Val
        35                  40                  45

Ala Ser Ile Asn Ser Asn Gly Gly Ser Thr Tyr Tyr Pro Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Ser Gly Asp Tyr Trp Gly Gln Gly Thr Thr Val Thr Val Ser Ser
            100                 105                 110

Ala Ser Thr Lys Gly Pro Ser Val Phe Pro Leu Ala Pro Cys Ser Arg
        115                 120                 125
```

Ser Thr Ser Glu Ser Thr Ala Ala Leu Gly Cys Leu Val Lys Asp Tyr
130 135 140

Phe Pro Glu Pro Val Thr Val Ser Trp Asn Ser Gly Ala Leu Thr Ser
145 150 155 160

Gly Val His Thr Phe Pro Ala Val Leu Gln Ser Ser Gly Leu Tyr Ser
165 170 175

Leu Ser Ser Val Val Thr Val Pro Ser Ser Ser Leu Gly Thr Lys Thr
180 185 190

Tyr Thr Cys Asn Val Asp His Lys Pro Ser Asn Thr Lys Val Asp Lys
195 200 205

Arg Val Glu Ser Lys Tyr Gly Pro Pro Cys Pro Pro Cys Pro Ala Pro
210 215 220

Glu Phe Leu Gly Gly Pro Ser Val Phe Leu Phe Pro Pro Lys Pro Lys
225 230 235 240

Asp Thr Leu Met Ile Ser Arg Thr Pro Glu Val Thr Cys Val Val Val
245 250 255

Asp Val Ser Gln Glu Asp Pro Glu Val Gln Phe Asn Trp Tyr Val Asp
260 265 270

Gly Val Glu Val His Asn Ala Lys Thr Lys Pro Arg Glu Glu Gln Phe
275 280 285

Asn Ser Thr Tyr Arg Val Val Ser Val Leu Thr Val Leu His Gln Asp
290 295 300

Trp Leu Asn Gly Lys Glu Tyr Lys Cys Lys Val Ser Asn Lys Gly Leu
305 310 315 320

Pro Ser Ser Ile Glu Lys Thr Ile Ser Lys Ala Lys Gly Gln Pro Arg
325 330 335

Glu Pro Gln Val Tyr Thr Leu Pro Pro Ser Gln Glu Glu Met Thr Lys
340 345 350

Asn Gln Val Ser Leu Thr Cys Leu Val Lys Gly Phe Tyr Pro Ser Asp
355 360 365

Ile Ala Val Glu Trp Glu Ser Asn Gly Gln Pro Glu Asn Asn Tyr Lys
370 375 380

Thr Thr Pro Pro Val Leu Asp Ser Asp Gly Ser Phe Phe Leu Tyr Ser
385 390 395 400

```
Arg Leu Thr Val Asp Lys Ser Arg Trp Gln Glu Gly Asn Val Phe Ser
                405                 410                 415

Cys Ser Val Met His Glu Ala Leu His Asn His Tyr Thr Gln Lys Ser
            420                 425                 430

Leu Ser Leu Ser Leu Gly Lys
        435
```

## Description

### Field of the invention

The present invention relates to novel compounds that can be employed in the treatment of a group of disorders and abnormalities associated with amyloid protein, such as Alzheimer's disease, and of diseases or conditions associated with amyloid-like proteins. The present invention further relates to pharmaceutical compositions comprising these compounds and to the use of these compounds for the preparation of medicaments for the treatment of diseases or conditions associated with amyloid or amyloid-like proteins. A method of treating diseases or conditions associated with amyloid or amyloid-like proteins is also disclosed.

The compounds of the present invention can also be used in the treatment of ocular diseases associated with pathological abnormalities/changes in the tissues of the visual system, particularly associated with amyloid-beta-related pathological abnormalities/changes in the tissues of the visual system, such as neuronal degradation. Said pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for examples age-related macular degeneration; the optic nerve leading to optic nerve drusen, optic neuropathy and optic neuritis; and the cornea leading to lattice dystrophy.

### Background of the invention

Many diseases of aging are based on or associated with amyloid or amyloid-like proteins and are characterized, in part, by the buildup of extracellular deposits of amyloid or amyloid-lie material that contribute to the pathogenesis, as well as the progression of the disease. These diseases include, but are not limited to, neurological disorders such as Alzheimer's disease (AD), diseases or conditions characterized by a loss of cognitive memory capacity such as, for example, mild cognitive impairment (MCI), Lewy body dementia, Down's syndrome, hereditary cerebral hemorrhage with amyloidosis (Dutch type); the Guam Parkinson-Dementia complex. Other diseases which are based on or associated with amyloid-like proteins are progressive supranuclear palsy, multiple sclerosis; Creutzfeld Jacob disease, Parkinson's disease, HIV-related dementia, ALS (amyotropic lateral sclerosis), inclusion-body myositis (IBM), Adult Onset Diabetes; senile cardiac amyloidosis; endocrine tumors, and other diseases, including amyloid-associated ocular diseases that target different tissues of the eye, such as the visual cortex, including cortical visual deficits; the anterior chamber and the optic nerve, including glaucoma; the lens, including cataract due to beta-amyloid deposition; the vitreous, including ocular amyloidosis; the retina, including primary retinal degenerations and macular degeneration, in particular age-related macular degeneration; the optic nerve, including optic nerve drusen, optic neuropathy and optic neuritis; and the cornea, including lattice dystrophy.

Although pathogenesis of these diseases may be diverse, their characteristic deposits often contain many shared molecular constituents. To a significant degree, this may be attributable to the local activation of pro-inflammatory pathways thereby leading to the concurrent deposition of activated complement components, acute phase reactants, immune modulators, and other inflammatory mediators.

Alzheimer's disease (AD) is a neurological disorder primarily thought to be caused by amyloid plaques, an accumulation of abnormal deposit of proteins in the brain. The most frequent type of amyloid found in the brain of affected individuals is composed primarily of Aβ fibrils. Scientific evidence demonstrates that an increase in the production and accumulation of beta-amyloid protein in plaques leads to nerve cell death, which contributes to the development and progression of AD. Loss of nerve cells in strategic brain areas, in turn, causes reduction in the neurotransmitters and impairment of memory. The proteins principally responsible for the plaque build up include amyloid precursor protein (APP) and two presenilins (presenilin I and presenilin II). Sequential cleavage of the amyloid precursor protein (APP), which is constitutively expressed and catabolized in most cells, by the enzymes β and γ secretase leads to the release of a 39 to 43 amino acid Aβ peptide. The degradation of APPs likely increases their propensity to aggregate in plaques. It is especially the Aβ(1-42) fragment that has a high propensity of building aggregates due to two very hydrophobic amino acid residues at its C-terminus. The Aβ(1-42) fragment is therefore believed to be mainly involved and responsible for the initiation of neuritic plaque formation in AD and to have, therefore, a high pathological potential. There is therefore a need for specific molecules that can target and diffuse amyloid plaque formation.

The symptoms of AD manifest slowly and the first symptom may only be mild forgetfulness. In this stage, individuals may forget recent events, activities, the names of familiar people or things and may not be able to solve simple math problems. As the disease progresses, symptoms are more easily noticed and become serious enough to cause people with AD or their family members to seek medical help. Mid-stage symptoms of AD include forgetting how to do simple tasks such as grooming, and problems develop with speaking, understanding, reading, or writing. Later stage AD patients may become anxious or aggressive, may wander away from home and ultimately need total care.

Presently, the only definite way to diagnose AD is to identify plaques and tangles in brain tissue in an autopsy after the death of the individual. Therefore, doctors can only make a diagnosis of "possible" or "probable" AD while the person is still alive. Using current methods, physicians can diagnose AD correctly up to 90 percent of the time using several tools to diagnose "probable" AD. Physicians ask questions about the person's general health, past medical problems, and the history of any difficulties the person has carrying out daily activities. Behavioral tests of memory, problem solving, attention, counting, and language provide information on cognitive degeneration and medical tests such as tests of blood, urine, or spinal fluid, and brain scans can provide some further information.

The management of AD consists of medication-based and non-medication based treatments. Treatments aimed at changing the underlying course of the disease (delaying or reversing the progression) have so far been largely unsuccessful. Medicines that restore the deficit (defect), or malfunctioning, in the chemical messengers of the nerve cells (neurotransmitters), in particular the cholinesterase inhibitors (ChEIs) such as tacrine and rivastigmine, have been shown to improve symptoms. ChEIs impede the enzymatic degradation of neurotransmitters thereby increasing the amount of chemical messengers available to transmit the nerve signals in the brain.

For some people in the early and middle stages of the disease, the drugs tacrine (COGNEX®, Morris Plains, NJ), donepezil (ARICEPT®, Tokyo, JP), rivastigmine (EXELON®, East Hanover, NJ), or galantamine (REMINYL®, New Brunswick, NJ) may help prevent some symptoms from becoming worse for a limited time. Another drug, memantine (NAMENDA®, New York, NY), has been approved for treatment of moderate to severe AD. Medications are also available to address the psychiatric manifestations of AD. Also, some medicines may help control behavioral symptoms of AD such as sleeplessness, agitation, wandering, anxiety, and depression. Treating these symptoms often makes patients more comfortable and makes their care easier for caregivers. Unfortunately, despite significant treatment advances showing that this class of agents is consistently better than a placebo, the disease continues to progress, and the average effect on mental functioning has only been modest. Many of the drugs used in AD medication such as, for example, ChEIs also have side effects that include gastrointestinal dysfunction, liver toxicity and weight loss.

Other diseases that are based on or associated with the accumulation and deposit of amyloid-like protein are mild cognitive impairment, Lewy body dementia (LBD), amyotrophic lateral sclerosis (ALS), inclusion-body myositis (IBM) and macular degeneration, in particular age-related macular degeneration (AMD).

Mild cognitive impairment (MCI) is a general term most commonly defined as a subtle but measurable memory disorder. A person with MCI experiences memory problems greater than normally expected with aging, but does not show other symptoms of dementia, such as impaired judgment or reasoning.

Lewy body dementia (LBD) is a neurodegenerative disorder that can occur in persons older than 65 years of age, which typically causes symptoms of cognitive (thinking) impairment and abnormal behavioral changes. Symptoms can include cognitive impairment, neurological signs, sleep disorder, and autonomic failure. Cognitive impairment is the presenting feature of LBD in most cases. Patients have recurrent episodes of confusion that progressively worsen. The fluctuation in cognitive ability is often associated with shifting degrees of attention and alertness. Cognitive impairment and fluctuations of thinking may vary over minutes, hours, or days.

Amyotrophic lateral sclerosis (ALS) is characterized by degeneration of upper and lower motor neurons. In some ALS patients, dementia or aphasia may be present (ALS-D). The dementia is most commonly a frontotemporal dementia (FTD), and many of these cases have ubiquitin-positive, tau-negative inclusions in neurons of the dentate gyrus and superficial layers of the frontal and temporal lobes.

Inclusion-body myositis (IBM) is a crippling disease usually found in people over age 50, in which muscle fibers develop inflammation and begin to atrophy - but in which the brain is spared and patients retain their full intellect. Two enzymes involved in the production of amyloid-ß protein were found to be increased inside the muscle cells of patients with this most common, progressive muscle disease of older people, in which amyloid-ß is also increased.

Macular degeneration is a common eye disease that causes deterioration of the macula, which is the central area of the retina (the paper-thin tissue at the back of the eye where light-sensitive cells send visual signals to the brain). Sharp, clear, 'straight ahead' vision is processed by the macula. Damage to the macula results in the development of blind spots and blurred or distorted vision. Age-related macular degeneration (AMD) is a major cause of visual impairment in the United States and for people over age 65 it is the leading cause of legal blindness among Caucasians. Approximately 1.8 million Americans age 40 and older have advanced AMD, and another 7.3 million people with intermediate AMD are at substantial risk for vision loss. The government estimates that by 2020 there will be 2.9 million people with advanced AMD. Victims of AMD are often surprised and frustrated to find out how little is known about the causes and treatment of this blinding condition.

There are two forms of macular degeneration: dry macular degeneration and wet macular degeneration. The dry form, in which the cells of the macula slowly begin to break down, is diagnosed in 85 percent of macular degeneration cases. Both eyes are usually affected by dry AMD, although one eye can lose vision while the other eye remains unaffected. Drusen, which are yellow deposits under the retina, are common early signs of dry AMD. The risk of developing advanced dry AMD or wet AMD increases as the number or size of the drusen increases. It is possible for dry AMD to advance and cause loss of vision without turning into the wet form of the disease; however, it is also possible for early-stage dry AMD to suddenly change into the wet form.

The wet form, although it only accounts for 15 percent of the cases, results in 90 percent of the blindness, and is considered advanced AMD (there is no early or intermediate stage of wet AMD). Wet AMD is always preceded by the dry form of the disease. As the dry form worsens, some people begin to have abnormal blood vessels growing behind the macula. These vessels are very fragile and will leak fluid and blood (hence 'wet' macular degeneration), causing rapid damage to the macula.

The dry form of AMD will initially often cause slightly blurred vision. The center of vision in particular may then become blurred and this region grows larger as the disease progresses. No symptoms may be noticed if only one eye is affected. In wet AMD, straight lines may appear wavy and central vision loss can occur rapidly.

Diagnosis of macular degeneration typically involves a dilated eye exam, visual acuity test, and a viewing of the back of the eye using a procedure called fundoscopy to help diagnose AMD, and - if wet AMD is suspected - fluorescein angiography may also be performed. If dry AMD reaches the advanced stages, there is no current treatment to prevent vision loss. However, a specific high dose formula of antioxidants and zinc may delay or prevent intermediate AMD from progressing to the advanced stage. Macugen® (pegaptanib sodium injection), laser photocoagulation and photodynamic therapy can control the abnormal blood vessel growth and bleeding in the macula, which is helpful for some people who have wet AMD; however, vision that is already lost will not be restored by these techniques. If vision is already lost, low vision aids exist that can help improve the quality of life.

One of the earliest signs of age-related macular degeneration (AMD) is the accumulation of extracellular deposits known as drusen between the basal lamina of the retinal pigmented epithelium (RPE) and Bruch's membrane (BM). Recent studies conducted by Anderson et al. have confirmed that drusen contains amyloid beta (Experimental Eye Research 78 (2004) 243-256).

Prions cause neurodegenerative diseases such as scrapie in sheep, bovine spongiform encephalopathy in cattle and Creutzfeldt-Jacob disease in humans. The only known component of the particle is the scrapie isoform of the protein, PrPSc. Although prions multiply, there is no evidence that they contain nucleic acid. PrPSc is derived from the non-infectious, cellular protein PrPC by a posttranslational process during which PrPC undergoes a profound conformational change.

The scrapie protein PrPSc has a critical role in neuronal degeneration and during disease development undergoes a three stage transition as follows: PrPC (normal cellular isoform of protein) - PrPSc: infectious form (scrapie isoform of protein) - protein PrP27-30.

Such a cascade of events occurs during the development of Creutzfeldt-Jacob disease (CJD), Kuru, Gerstmann-Straussler-Scheinker Syndrome (GSS), fatal familial insomnia in man, scrapie in sheep and goats, encephalopathy in mink and bovine spongiform encephalopathy in cattle.

The cellular non-toxic protein (PrPC) is a sialoglycoprotein of molecular weight 33000 to 35000 that is expressed predominantly in neurons. In the diseases mentioned above, PrPC is converted into an altered form (PrPSc), which is distinguishable from its normal homologue by its relative resistance to protease digestion. PrPSc accumulates in the central nervous system of affected animals and individuals and its protease-resistant core aggregates extracellularly.

Amyloidosis is not a single disease entity but rather a diverse group of progressive disease processes characterized by extracellular tissue deposits of a waxy, starch-like protein called amyloid, which accumulates in one or more organs or body systems. As the amyloid deposits build up, they begin to interfere with the normal function of the organ or body system. There are at least 15 different types of amyloidosis. The major forms are primary amyloidosis without known antecedent, secondary amyloidosis following some other condition, and hereditary amyloidosis.

Secondary amyloidosis occurs in people who have a chronic infection or inflammatory disease, such as tuberculosis, a bacterial infection called familial Mediterranean fever, bone infections (osteomyelitis), rheumatoid arthritis, inflammation of the small intestine (granulomatous ileitis), Hodgkin's disease, and leprosy.

Glaucoma is a group of diseases of the optic nerve involving loss of retinal ganglion cells (RGCs) in a characteristic pattern of optic neuropathy. Glaucoma is often, but not always, accompanied by an increased eye pressure, which may be a result of blockage of the circulation of aqueous, or its drainage.

Although raised intraocular pressure is a significant risk factor for developing glaucoma, no threshold of intraocular pressure can be defined which would be determinative for causing glaucoma.

The damage may also be caused by poor blood supply to the vital optic nerve fibers, a weakness in the structure of the nerve, and/or a problem in the health of the nerve fibers themselves.

Untreated glaucoma leads to permanent damage of the optic nerve and resultant visual field loss, which can progress to blindness.

RGCs are the nerve cells that transmit visual signals from the eye to the brain. Caspase-3 and Caspase-8, two major enzymes in the apoptotic process, are activated in the process leading to apoptosis of RGCs. Caspase-3 cleaves amyloid precursor protein (APP) to produce neurotoxic fragments, including Amyloid β. Without the protective effect of APP, Amyloid β accumulation in the retinal ganglion cell layer results in the death of RGCs and irreversible loss of vision.

The different types of glaucomas are classified as open-angle glaucomas, if the condition is chronic, or closed-angle glaucomas, if acute glaucoma occurs suddenly. Glaucoma usually affects both eyes, but the disease can progress more rapidly in one eye than in the other.

Chronic open-angle glaucoma (COAG), also known as primary open angle glaucoma (POAG), is the most common type of glaucoma. COAG is caused by microscopic blockage in the trabecular meshwork, which decreases the drainage of the aqueous outflow into the Schlemm's canal and raises the intraocular pressure (IOP). POAG usually affects both eyes and is strongly associated with age and a positive family history. Its frequency increases in elderly people as the eye drainage mechanism may gradually become clogged with aging. The increase in intraocular pressure in subjects affected by chronic open-angle glaucoma is not accompanied by any symptoms until the loss is felt on the central visual area.

Acute Angle Closure Glaucoma (AACG) or closed-angle glaucoma is a relatively rare type of glaucoma characterized by a sudden increase in intraocular pressure to 35 to 80 mmHg, leading to severe pain and irreversible loss of vision. The sudden pressure increase is caused by the closing of the filtering angle and blockage of the drainage channels. Individuals with narrow angles have an increased risk for a sudden closure of the angle. AACG usually occurs monocularly, but the risk exists in both eyes. Age, cataract and pseudoexfoliation are also risk factors since they are associated with enlargement of the lens and crowding or narrowing of the angle. A sudden glaucoma attack may be associated with severe eye pain and headache, inflamed eye, nausea, vomiting, and blurry vision.

Mixed or Combined Mechanism Glaucoma is a mixture or combination of open and closed angle glaucoma. It affects patients with acute ACG whose angle opens after laser iridotomy, but who continue to require medications for IOP control, as well as patients with POAG or pseudoexfoliative glaucoma who gradually develop narrowing of the angle.

Normal tension glaucoma (NTG), also known as low tension glaucoma (LTG), is characterized by progressive optic nerve damage and loss of peripheral vision similar to that seen in other types of glaucoma; however, the intraocular pressure is in the normal range or even below normal.

Congenital (infantile) glaucoma is a relatively rare, inherited type of open-angle glaucoma. Insufficient development of the drainage area results in increased pressure in the eye that can lead to the loss of vision from optic nerve damage and to an enlarged eye. Early diagnosis and treatment are critical to preserve vision in infants and children affected by the disease.

Secondary glaucoma may result from an ocular injury, inflammation in the iris of the eye (iritis), diabetes, cataract, or use of steroids in steroid-susceptible individuals. Secondary glaucoma may also be associated with retinal detachment or retinal vein occlusion or blockage.

Pigmentary glaucoma is characterized by the detachment of granules of pigment from the iris. The granules cause blockage of the drainage system of the eye, leading to elevated intraocular pressure and damage to the optic nerve.

Exfoliative glaucoma (pseudoexfoliation) is characterized by deposits of flaky material on the anterior capsule and in the angle of the eye. Accumulation of the flaky material blocks the drainage system and raises the eye pressure.

Diagnosis of glaucoma may be made using various tests. Tonometry determines the pressure in the eye by measuring the tone or firmness of its surface. Several types of tonometers are available for this test, the most common being the applanation tonometer. Pachymetry determines the thickness of the cornea which, in turn, measures intraocular pressure. Gonioscopy allows examination of the filtering angle and drainage area of the eye. Gonioscopy can also determine if abnormal blood vessels may be blocking the drainage of the aqueous fluid out of the eye. Ophthalmoscopy allows examination of the optic nerve and can detect nerve fiber layer drop or changes in the optic disc, or indentation (cupping) of this structure, which may be caused by increased intraocular pressure or axonal drop out. Gonioscopy is also useful in assessing damage to the nerve from poor blood flow or increased intraocular pressure. Visual field testing maps the field of vision, subjectively, which may detect signs of glaucomatous damage to the optic nerve. This is represented by specific patterns of visual field loss. Ocular coherence tomography, an objective measure of nerve fiber layer loss, is carried out by looking at the thickness of the optic nerve fiber layer (altered in glaucoma) via a differential in light transmission through damaged axonal tissue.

Optic nerve drusen are globular concretions of protein and calcium salts which are felt to represent secretions through congenitally altered vascular structures affecting the axonal nerve fiber layer. These accumulations occur in the peripapillary nerve fiber layer and are felt to damage the nerve fiber layer either directly by compression or indirectly through disruptions of the vascular supply to the nerve fiber layer. They usually become visible after the first decade of life in affected individuals. They occur most often in both eyes but may also affect one eye, and may cause mild loss of peripheral vision over many years.

Optic neuropathy is a disease characterized by damage to the optic nerve caused by demyelination, blockage of blood supply, nutritional deficiencies, or toxins. Demyelinating optic neuropathies (see optic neuritis below) are typically caused by an underlying demyelinating process such as multiple sclerosis. Blockage of the blood supply, known as ischemic optic neuropathy, can lead to death or dysfunction of optic nerve cells. Non-arteritic ischemic optic neuropathy usually occurs in middle-age people. Risk factors include high blood pressure, diabetes and atherosclerosis. Arteritic ischemic optic neuropathy usually occurs in older people following inflammation of the arteries (arteritis), particularly the temporal artery (temporal arteritis). Loss of vision may be rapid or develop gradually over 2 to 7 days and the damage

may be to one or both eyes. In people with optic neuropathy caused by exposure to a toxin or to a nutritional deficiency, both eyes are usually affected.

About 40% of people with non-arteritic ischemic optic neuropathy experience spontaneous improvement over time. Non-arteritic ischemic optic neuropathy is treated by controlling blood pressure, diabetes and cholesterol levels. Arteritic ischemic optic neuropathy is treated with high doses of corticosteroids to prevent loss of vision in the second eye.

Optic neuritis is associated with mild or severe vision loss in one or both eyes and may be caused by a systemic demyelinating process (see above), viral infection, vaccination, meningitis, syphilis, multiple sclerosis and intraocular inflammation (uveitis). Eye movement may be painful and vision may deteriorate with repeat episodes. Diagnosis involves examination of the reactions of the pupils and determining whether the optic disk is swollen. Magnetic resonance imaging (MRI) may show evidence of multiple sclerosis or, rarely, a tumor pressing on the optic nerve, in which case vision improves once the tumor pressure is relieved. Most cases of optic neuritis improve over a few months without treatment. In some cases, treatment with intravenous corticosteroids may be necessary.

A cataract is an opacity that develops in the crystalline lens of the eye or in its envelope. Cataracts typically cause progressive vision loss and may cause blindness if left untreated. In the Morgagnian Cataract, the cataract cortex progressively liquefies to form a milky white fluid and may cause severe inflammation if the lens capsule ruptures and leaks. If left untreated, the cataract may also cause phacomorphic glaucoma. Cataracts may be congenital in nature or caused by genetic factors, advanced age, long-term ultraviolet exposure, exposure to radiation, diabetes, eye injury or physical trauma.

Extra-capsular (ECCE) surgery is the most effective treatment to treat cataract. In the surgery, the lens is removed, but the majority of the lens capsule is left intact. Phacoemulsification, a small incision on the side of the cornea, is typically used to break up the lens before extraction.

Ocular amyloidosis is a hereditary disorder associated with Type I Familial Amyloidotic Polyneuropathy (FAP) and characterized by abnormal conjunctival vessels, keratoconjunctivitis sicca, pupillary abnormalities and, in some cases, vitreous opacities and secondary glaucoma. Type I FAP is associated with mutations in transthyretin (TTR), a tetrameric plasma protein (prealbumin) synthesized in the liver, the retinal pigment epithelium2 and thechoroid plexus of the brain. Different mutations cause transthyretin to polymerize into a pleated structure of amyloid fibril, leading to hereditary amyloidosis. The most frequent mutation is TTR-met303, in which methionine replaces valine at position 30 in transthyretin.

Type IV FAP is associated with lattice corneal dystrophy (LCD). Lattice corneal dystrophy is an inherited, primary, usually bilateral corneal amyloidosis characterized by the presence of refractile lattice lines with a double contour in the corneal stroma. LCD type I (Biber-Haab-Dimmer) is an autosomal dominant, bilaterally symmetrical corneal disorder characterized by the presence of numerous translucent fine lattice lines with white dots and faint haze in the superficial and middle layers of the central stroma. The symptoms start during the first or second decades of life, causing a progressive loss of vision. Most patients require a corneal transplant by 40 years of age. LCD type II is associated with systemic amyloidosis (Meretoja's syndrome) and is characterized by the presence of thick lattice lines in the limbus, central cornea and stroma. Vision is not affected until later in life. LCD type III affects middle-age people and is characterized by the presence of thick lattice lines that extend from limbus to limbus. LCD type III A is characterized by the accumulation of amyloid deposits in the stroma and the presence of ribbons of amyloid between the stroma and Bowman's layer. LCD type III A differs from LCD type III because of the presence of corneal erosions, the occurrence in whites and the autosomal dominant inheritance pattern.

Down's Syndrome (DS) or trisomy 21 is the most common genetic disorder with an incidence of about 1:700 live births, and is often associated with various congenital anomalies. The disorder, which is caused by the presence of an extra chromosome 21, is associated with premature deposits of the plaque-forming protein amyloid-beta and development of Alzheimer's disease by middle age. Furthermore, many people affected by DS suffer from cataracts beginning in childhood and many suffer from congenital glaucoma. Since the gene for amyloid precursor protein, which is cleaved to form amyloid beta, is located on the long arm of chromosome 21 in humans, overexpression of this gene may lead to increased levels of amyloid precursor protein and amyloid deposition in Down's syndrome.

There is no cure for glaucoma. Medications for the treatment of glaucoma include agents that decrease production of the aqueous humor in the eye, such as beta blockers (Timoptic, Betoptic), carbonic anhydrase inhibitors (Trusopt, Azopt), and alpha agonists (Alphagan, lopidine), and agents that redirect drainage of the aqueous humor through a different pathway at the back of the eye, such as prostaglandin (Xalatan). Laser surgeries include trabeculoplasty, a procedure that helps the aqueous humor leave the eye more efficiently. According to the Glaucoma Foundation, nearly 80% of the patients respond well enough to the procedure to delay or avoid further surgery. However, pressure increases again in the eyes of half of all patients within two years after laser surgery, according to the National Eye Institute. Incisional surgery is performed if medication and initial laser treatments are unsuccessful in reducing pressure within the eye. One type of surgery, a trabeculectomy, creates an opening in the wall of the eye so that aqueous humor can drain. However, about one-third of trabeculectomy patients develop cataracts within five years, according to the Glaucoma Foundation. If the trabeculectomy fails, additional incisional procedures include placing a drainage tube into

the eye between the cornea and iris and the use of a laser or freezing treatment to destroy tissue in the eye that makes aqueous humor. Surgery may save the remaining vision in the patient, but it does not improve sight. Vision may actually be worse following surgery.

Age-related macular degeneration (AMD) is a major cause of blindness among Caucasians over age 65. Although much progress has been made recently in macular degeneration research, there are no treatments that rescue neuronal cell death that occurs during the course of the disease. There are also no definitive treatments for other ocular diseases associated with amyloid beta-related neuronal degradation, such as cortical visual deficits, optic nerve drusen, optic neuropathy, optic neuritis, ocular amyloidosis and lattice dystrophy.

Amyloid deposits typically contain three components. Amyloid protein fibrils, which account for about 90% of the amyloid material, comprise one of several different types of proteins. These proteins are capable of folding into so-called "beta-pleated" sheet fibrils, a unique protein configuration which exhibits binding sites for Congo red resulting in the unique staining properties of the amyloid protein. In addition, amyloid deposits are closely associated with the amyloid P (pentagonal) component (AP), a glycoprotein related to normal serum amyloid P (SAP), and with sulphated glycosaminoglycans (GAG), complex carbohydrates of connective tissue.

One development towards the treatment of Alzheimer's disease and prion diseases has been the design of molecules that bind the abnormal β-sheep conformation of Aβ and PrP, respectively, thereby preventing aggregation of these molecules. The β-sheet conformation of peptides is characterized in that hydrogen bonds are formed in a regular pattern between neighboring amino acid strands. This arrangement leads to a stable three dimensional structure. H-bond acceptors (C=O group) and H-bond donors (NH group) alternate in naturally occurring β-sheet peptides with the atoms to be bonded being roughly in one line. Within each amino acid strand, the distances between neighboring H-bond donors and H-bond acceptors fall within specific ranges. In particular, the distance between the H-bond donor (NH group) and the H-bond acceptor (C=O group) within one amino acid residue is from 3.5 to 4.0 A. The distance between the H-bond acceptor (C=O group) of one amino acid residue and the H-bond donor (NH group) of the following amino acid residue participating in the inter-strand bonding is from 2.6 to 2.9 A. In other words, the distances between neighboring H-bond donors and H-bond acceptors within one amino acid strand alternate between the following ranges:

H-bond donor (amino acid 1) - H-bond acceptor (amino acid 1) = 3.5 to 4.0 Å;
H-bond acceptor (amino acid 1) - H-bond donor 2 (amino acid 2) = 2.6 to 2.9 Å.

Ligands that are designed to bind β-sheets ideally have an order of H-bond donors and H-bond acceptors that is complementary to the order of H-bond donors and H-bond acceptors in the amino acid strands of the β-sheet.

It was an object of the present invention to provide compounds that can be employed in the treatment of diseases or conditions associated with amyloid or amyloid-like proteins, including amyloidosis. The compounds should be able to pass the blood-brain barrier. Furthermore, they should be pharmaceutically acceptable, in particular, they should not have mutagenic or carcinogenic properties or be metabolically unstable.

A further object of the invention is to provide improved treatment options for subjects affected by ocular diseases associated with pathological abnormalities/changes in the tissues of the visual system, particularly associated with amyloid-beta-related pathological abnormalities/changes in the tissues of the visual system, such as, for example, neuronal degradation. Said pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy.

The present inventors have found that these objects can be achieved by the compounds of the general formula (I) as described hereinafter.

**Summary of the invention**

Accordingly, the present invention relates to a compound of general formula (I).

In a further aspect, the present invention relates to a pharmaceutical composition comprising a compound of general formula (I).

Yet another aspect of the present invention relates to the use of a compound of general formula (I) for the preparation of a medicament for the treatment of diseases or conditions associated with amyloid or amyloid-like proteins, including amyloidosis.

Also disclosed herein is a method of treating diseases or conditions associated with amyloid or amyloid-like proteins, comprising administering to a subject in need of such treatment an effective amount of a compound of general formula (I).

Yet another aspect of the present invention relates to the use of a compound of general formula (I) for the

preparation of a medicament for treating or alleviating the effects of ocular diseases associated with pathological abnormalities/changes in the tissues of the visual system.

Also disclosed herein is a method of treating or alleviating the effects of ocular diseases associated with pathological abnormalities/changes in the tissues of the visual system comprising administering to a subject in need of such treatment an effective amount of a compound of general formula (I).

The ocular diseases associated with pathological abnormalities/changes in the tissues of the visual system are particularly associated with amyloid-beta-related pathological abnormalities/changes in the tissues of the visual system, such as, for example, neuronal degradation. Said pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy.

In a further aspect the invention relates to a mixture (such as a pharmaceutical composition) comprising a compound according to the present invention and optionally at least one further biologically active compound and/or a pharmaceutically acceptable carrier and/or a diluent and/or an excipient. The further biologically active substance can be a known compound used in the medication of diseases and disorders which are caused by or associated with amyloid or amyloid-like proteins including amyloidosis, a group of diseases and disorders associated with amyloid or amyloid-like protein such as the Aβ protein involved in Alzheimer's disease.

The further biologically active substance or compound may exert its biological effect by the same or a similar mechanism as the compound according to the invention or by an unrelated mechanism of action or by a multiplicity of related and/or unrelated mechanisms of action.

A method of collecting data for the diagnosis of an amyloid-associated disease or condition in a sample or a patient is also disclosed which comprises:

(a) bringing a sample or a specific body part or body area suspected to contain an amyloid protein into contact with a compound according to the present invention;
(b) allowing the compound to bind to the amyloid protein;
(c) detecting the compound bound to the protein; and
(d) optionally correlating the presence or absence of compound binding with the amyloid protein with the presence or absence of amyloid protein in the sample or specific body part or area.

Another embodiment of the present invention is a method of determining the extent of amyloidogenic plaque burden in a tissue and/or a body fluid comprising:

(a) providing a sample representative of the tissue and/or body fluid under investigation;
(b) testing the sample for the presence of amyloid protein with a compound according to the present invention;
(c) determining the amount of compound bound to the amyloid protein; and
(d) calculating the plaque burden in the tissue and/or body fluid.

A further aspect relates to a method of collecting data for determining a predisposition to an amyloid-associated disease or condition in a patient comprising detecting the specific binding of a compound according to the present invention to an amyloid protein in a sample or in situ which comprises the steps of:

(a) bringing the sample or a specific body part or body area suspected to contain the amyloid protein into contact with a compound according to the present invention, which compound specifically binds to the amyloid protein;
(b) allowing the compound to bind to the amyloid protein to form a compound/protein complex;
(c) detecting the formation of the compound/protein complex;
(d) optionally correlating the presence or absence of the compound/protein complex with the presence or absence of amyloid protein in the sample or specific body part or area; and
(e) optionally comparing the amount of the compound/protein complex to a normal control value.

Yet another aspect of the present invention is a method of collecting data for monitoring minimal residual disease in a patient following treatment with an antibody or a vaccine composition, wherein the method comprises:

(a) bringing a sample or a specific body part or body area suspected to contain an amyloid protein into contact with a compound according to the present invention, which compound specifically binds to the amyloid protein;
(b) allowing the compound to bind to the amyloid protein to form a compound/protein complex;

(c) detecting the formation of the compound/protein complex;
(d) optionally correlating the presence or absence of the compound/protein complex with the presence or absence of amyloid protein in the sample or specific body part or body area; and
(e) optionally comparing the amount of the compound/protein complex to a normal control value.

A method of collecting data for predicting responsiveness of a patient being treated with an antibody or a vaccine composition is also described which comprises:

(a) bringing a sample or a specific body part or body area suspected to contain an amyloid protein into contact with a compound according to the present invention, which compound specifically binds to the amyloid protein;
(b) allowing the compound to bind to the amyloid protein to form a compound/protein complex;
(c) detecting the formation of the compound/protein complex;
(d) optionally correlating the presence or absence of the compound/protein complex with the presence or absence of amyloid protein in the sample or specific body part or body area; and
(e) optionally comparing the amount of the compound/protein complex to a normal control value.

A further aspect of the present invention is a test kit for detection and diagnosis of an amyloid-associated disease or condition comprising a compound according to the present invention.
In another aspect of the present invention a compound according to the present invention is for use in inhibiting protein aggregation, in particular for use in inhibiting Aβ1-42 aggregation, Tau aggregation or alpha-synuclein aggregation.

**Definitions**

Within the meaning of the present application the following definitions apply:

"alkyl" refers to a saturated organic moiety consisting of carbon and hydrogen atoms. Examples of suitable alkyl groups have 1 to 6 carbon atoms, preferably 1 to 4 carbon atoms, and include methyl, ethyl, propyl and butyl.

"Cycloalkyl" refers to a cyclic organic moiety consisting of carbon and hydrogen atoms. Examples of suitable alkyl groups have 5 to 10 carbon atoms, preferably 5 or 6 carbon atoms, and include cyclopentyl and cyclohexyl.

"Heterocycloalkyl" refers to a cycloalkyl group as defined above in which at least one of the carbon atoms has been replaced by a heteroatom which is, e.g., selected from N, O or S, or heteroatom (e.g., N, O and/or S)-containing moiety. Examples of possible heterocycloalkyl groups include pyrrolidine, tetrahydrofuran, piperidine, etc.

"Alkenyl" refers to an organic moiety consisting of carbon and hydrogen atoms which includes at least one double bond. Examples of suitable alkenyl groups have 2 to 6 carbon atoms, preferably 2 to 4 carbon atoms, and include propenyl and butenyl.

"Alkynyl" refers to an organic moiety consisting of carbon and hydrogen atoms which includes at least one triple bond. Examples of suitable alkinyl groups have 2 to 6 carbon atoms, preferably 2 to 4 carbon atoms, and include propinyl and butinyl.

"Aryl" refers to an aromatic organic moiety consisting of carbon and hydrogen atoms which preferably has 5 to 10 carbon atoms, more preferably 5 or 6 carbon atoms. An example is a phenyl ring.

"Heteroaryl" refers to an aryl group as defined above in which at least one of the carbon atoms has been replaced by a heteroatom which is, e.g., selected from N, O or S, or heteroatom (e.g., N, O and/or S)-containing moiety. Examples of possible heteroaryl groups include pyridine, etc.

"Alkoxy" refers to the group -O-alkyl.

"Aminoalkyl" refers to the group -alkyl-$NR^1R^2$.

"Hal" or "halogen" refers to F, Cl, Br, and I. Preferred Hal are F and Cl, more preferably F.

"Arylalkyl" refers to a group aryl-alkyl-.

"Cycloalkylalkyl" refers to a group cycloalkyl-alkyl-.

"Fluoroalkyl" refers to an alkyl group in which one or more hydrogen atoms have been replaced by fluoro atoms.

"Haloalkyl" refers to an alkyl group in which one or more hydrogen atoms have been replaced by halogen atoms.

"Heteroarylalkyl" refers to a group heteroaryl-alkyl-.

"Heterocycloalkylalkyl" refers to a group heterocycloalkyl-alkyl-.

"Heteroatom-containing moieties are moieties which contain e.g., N, O and/or S. Examples of such moieties include -C(O)-, -C(O)O- and -N($R^{50}$)- in which $R^{50}$ is, for each occurrence, independently selected from the group consisting of $R^{20}$, $S(O)_tNR^{20}R^{21}$, $S(O)_tR^{20}$, $C(O)OR^{20}$, $C(O)R^{20}C(=NR^a)NR^{20}R^{21}$, $C(=NR^{20})NR^{21}R^a$, $C(=NOR^{20})R^{21}$ and C (O)$NR^{20}R^{21}$. Specific examples include -C(O)-, -C(O)O- and -N($R^{50}$)- in which $R^{50}$ is, for each occurrence, independently selected from the group consisting of H or $C_{1-4}$ alkyl.

If a group is defined as being "optionally substituted" it can have one or more substituents selected from Hal, $C_{1-6}$ alkyl or $C_{1-6}$ alkoxy.

Compounds of the present invention having one or more optically active carbons can exist as racemates and racemic mixtures, stereoisomers (including diastereomeric mixtures and individual diastereomers, enantiomeric mixtures and single enantiomers, mixtures of conformers and single conformers), tautomers, atropisomers, and rotamers. All isomeric forms are included in the present invention. Compounds described in this invention containing olefinic double bonds include E and Z geometric isomers. Also included in this invention are all salt forms, polymorphs, hydrates and solvates.

The solvent included in the solvates is not particularly limited and can be any pharmaceutically acceptable solvent. Examples include water and $C_{1-4}$ alcohols.

The preferred definitions given in the "Definition"-section apply to all of the embodiments described below unless stated otherwise.

## Detailed description of the invention

The present invention relates to a compound of formula (I):

$$A\text{-}L_1\text{-}B \qquad (I)$$

and all stereoisomers, racemic mixtures, pharmaceutically acceptable salts, hydrates, solvates and polymorphs thereof.

A is selected from the group consisting of:

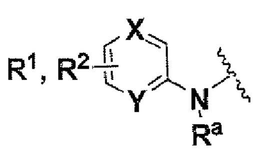
(II)
,
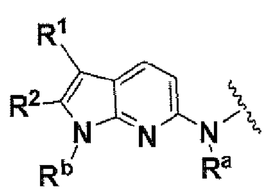
(III)
,
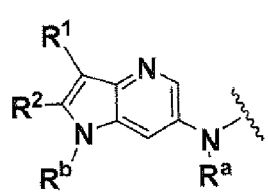
(IV)
,
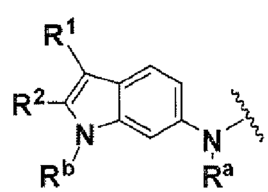
(V)

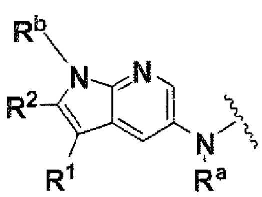
(VI)
,
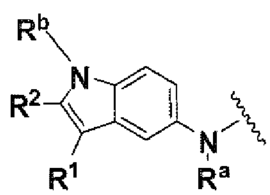
(VII)
,
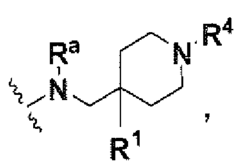
(VIII)
,
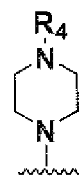
(IX)
or
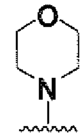
(X)

Preferably A is selected from the group consisting of formulae (II), (III) and (V).

$L_1$ is

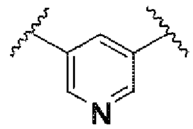

(XI)

B is selected from the group consisting of:

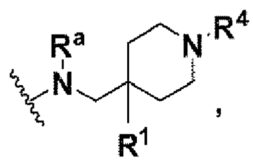

,

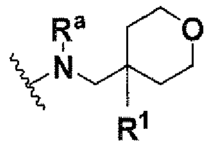

,

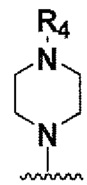

or

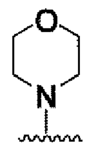

(XII) (XIII) (XIV) (XV)

Preferably B has the formula (XII).

$R^1$ is each independently selected from the group consisting of hydrogen, halogen, CN, $CF_3$, $CONR^4R^5$, alkyl, cycloalkyl, cycloalkylalkyl, heterocycloalkyl, fluoroalkyl, heterocycloalkylalkyl, alkenyl, alkynyl, aryl, heteroaryl, arylalkyl and heteroarylalkyl, wherein alkyl, cycloalkyl, cycloalkylalkyl, heterocycloalkyl, fluoroalkyl, heterocycloalkylalkyl, alkenyl, alkynyl, aryl, heteroaryl, arylalkyl and heteroarylalkyl can be optionally substituted. In a preferred embodiment $R^1$ is each independently selected from hydrogen, halogen (such as F or Cl, e.g., F), CN, fluoroalkyl (such as $CF_3$), and heterocycloalkyl (such as

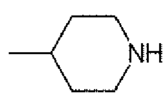

).

More preferably $R^1$ is each independently selected from hydrogen, F, $CF_3$ and

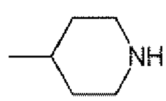

.

$R^2$ is each independently selected from the group consisting of hydrogen, halogen, CN, $CF_3$, $CONR^4R^5$, alkyl, cycloalkyl, cycloalkylalkyl, heterocycloalkyl, fluoroalkyl, heterocycloalkylalkyl, alkenyl, alkynyl, aryl, heteroaryl, arylalkyl and heteroarylalkyl, wherein alkyl, cycloalkyl, cycloalkylalkyl, heterocycloalkyl, fluoroalkyl, heterocycloalkylalkyl, alkenyl, alkynyl, aryl, heteroaryl, arylalkyl and heteroarylalkyl can be optionally substituted. In a preferred embodiment $R^2$ is each independently selected from hydrogen, halogen (such as F or Cl, e.g., F), CN, and fluoroalkyl (such as $CF_3$). More preferably $R^2$ is each independently selected from hydrogen and F.

In a further embodiment $R^1$ and $R^2$ when taken together can form a 5- to 8-membered ring containing carbon atoms and optionally one or more heteroatoms selected from O, S, or N or a heteroatom (N, O and/or S)-containing moiety wherein the 5- to 8-membered ring may be substituted by $NR^{20}R^{21}$. In one embodiment $R^1$ and $R^2$ when taken together can form a 5- to 8-membered ring containing carbon atoms such as a 6-membered carbocyclic ring. The ring can be saturated or include one or more double bonds (including aromatic rings).

$R^a$ is each independently selected from the group consisting of hydrogen, alkyl, haloalkyl, $S(O)_tNR^4R^5$, $S(O)_tR^4$, $C(O)OR^4$, $C(O)R^4$, and $C(O)NR^4R^5$. In a preferred embodiment $R^a$ is hydrogen or $C_{1-4}$ alkyl (e.g. methyl) or hydrogen.

$R^b$ is each independently selected from the group consisting of hydrogen, alkyl or haloalkyl. In a preferred embodiment $R^b$ is hydrogen or $C_{1-4}$ alkyl (e.g. methyl).

For each occurrence $R^3$ is each independently selected from the group consisting of: hydrogen, alkyl, cycloalkyl, cycloalkylalkyl, heterocycloalkyl, fluoroalkyl, heterocycloalkylalkyl, alkenyl, alkynyl, aryl, heteroaryl, arylalkyl, heteroarylalkyl and aminoalkyl, wherein alkyl, cycloalkyl, cycloalkylalkyl, heterocycloalkyl, fluoroalkyl, heterocycloalkylalkyl, alkenyl, alkynyl, aryl, heteroaryl, arylalkyl, heteroarylalkyl and aminoalkyl can be optionally substituted. In a preferred embodiment $R^3$ is hydrogen, halogen (such as F) or alkyl. More preferably $R^3$ is hydrogen or halogen (such as F).

For each occurrence $R^4$ is each independently selected from the group consisting of: hydrogen, alkyl, cycloalkyl, cycloalkylalkyl, heterocycloalkyl, fluoroalkyl, heterocycloalkylalkyl, alkenyl, alkynyl, aryl, heteroaryl, arylalkyl, heteroarylalkyl and aminoalkyl, wherein alkyl, cycloalkyl, cycloalkylalkyl, heterocycloalkyl, fluoroalkyl, heterocycloalkylalkyl, alkenyl, alkynyl, aryl, heteroaryl, arylalkyl, heteroarylalkyl and aminoalkyl can be optionally substituted. In a preferred embodiment $R^4$ is hydrogen or alkyl.

For each occurrence $R^5$ is each independently selected from the group consisting of: hydrogen, alkyl, cycloalkyl, cycloalkylalkyl, heterocycloalkyl, fluoroalkyl, heterocycloalkylalkyl, alkenyl, alkynyl, aryl, heteroaryl, arylalkyl, heteraarytalkyl- and aminoalkyl, wherein alkyl, cycloalkyl, cycloalkylalkyl, heterocycloalkyl, fluoroalkyl, heterocycloalkylalkyl, alkenyl, alkynyl, aryl, heteroaryl, arylalkyl, heteroarylalkyl and aminoalkyl can be optionally substituted. In a preferred embodiment $R^5$ is hydrogen or alkyl.

For each occurrence $R^{20}$ is each independently selected from the group consisting of: hydrogen, alkyl, cycloalkyl, cycloalkylalkyl, heterocycloalkyl, fluoroalkyl, heterocycloalkylalkyl, alkenyl, alkynyl, aryl, heteroaryl, arylalkyl, heteroarylalkyl and aminoalkyl, wherein alkyl, cycloalkyl, cycloalkylalkyl, heterocycloalkyl, fluoroalkyl, heterocycloalkylalkyl, alkenyl, alkynyl, aryl, heteroaryl, arylalkyl, heteroarylalkyl and aminoalkyl can be optionally substituted. In a preferred embodiment $R^{20}$ is hydrogen or alkyl.

For each occurrence $R^{21}$ is each independently selected from the group consisting of: hydrogen, alkyl, cycloalkyl, cycloalkylalkyl, heterocycloalkyl, fluoroalkyl, heterocycloalkylalkyl, alkenyl, alkynyl, aryl, heteroaryl, arylalkyl, heteroarylalkyl and aminoalkyl, wherein alkyl, cycloalkyl, cycloalkylalkyl, heterocycloalkyl, fluoroalkyl, heterocycloalkylalkyl, alkenyl, alkynyl, aryl, heteroaryl, arylalkyl, heteroarylalkyl and aminoalkyl can be optionally substituted. In a preferred embodiment $R^{21}$ is hydrogen or alkyl.

In one embodiment $R^{20}$ and $R^{21}$ when taken together with the nitrogen to which they are attached can form a 3- to 8-membered ring containing carbon atoms and optionally one or more further heteroatoms selected from O, S, or N or a heteroatom (N, O and/or S)-containing moiety and wherein the 3- to 8-membered ring may be optionally substituted. The ring can be saturated or include one or more double bonds (including aromatic rings). In one embodiment the ring is carbocyclic apart from the nitrogen atom to which $R^{20}$ and $R^{21}$ are attached. In a different embodiment the ring includes one further heteroatom (such as N or O) in addition to the nitrogen atom to which $R^{20}$ and $R^{21}$ are attached.

When a nitrogen atom is present in the ring formed by $R^1$ and $R^2$ or the ring formed by $R^{20}$ and $R^{21}$, it can be in any suitable form. Possible forms include $-N(R^{50})-$, and $-N=$.

$R^{50}$ is, for each occurrence, independently selected from the group consisting of $R^{20}$, $S(O)_tNR^{20}R^{21}$, $S(O)_tR^{20}$, $C(O)OR^{20}$, $C(O)R^{20}C(=NR^a)NR^{20}R^{21}$, $C(=NR^{20})NR^{21}R^a$, $C(=NOR^{20})R^{21}$ and $C(O)NR^{20}R^{21}$. In a preferred embodiment $R^{50}$ is hydrogen.

X is each independently selected from the group consisting of $CR^3$ and N.

Y is each independently selected from the group consisting of $CR^3$ and N.

t is 1 or 2.

Any combination of the above mentioned definitions is also envisaged in the present specification.

In one embodiment A has the formula (i)

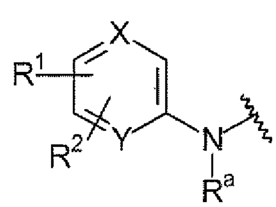

(II)

Preferred embodiments of the formula (II) include

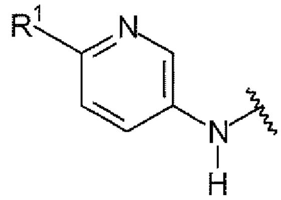

(in this embodiment $R^1$ is as defined above. In one embodiment $R^1$ is e.g. $CF_3$)

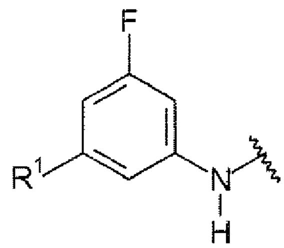

(In this embodiment $R^1$ is as defined above. In one embodiment $R^1$ is e.g. F)

In one embodiment A has the formula (III)

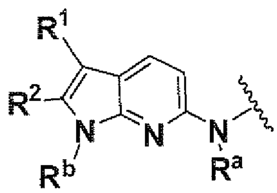

(III)

Preferred embodiments of the formula (III) include

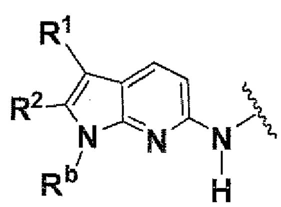

(wherein $R^1$, $R^2$ and $R^b$ are as defined above).

Preferred embodiments include

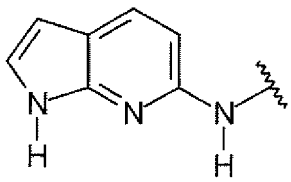

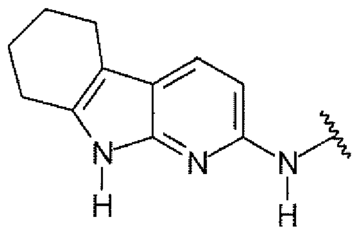

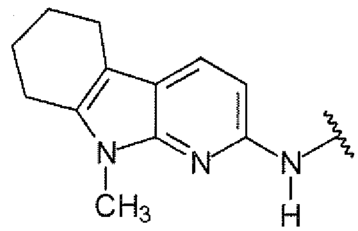

In one embodiment A has the formula (V)

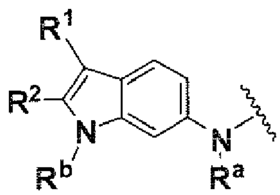

(V)

Preferred embodiments of the formula (V) include

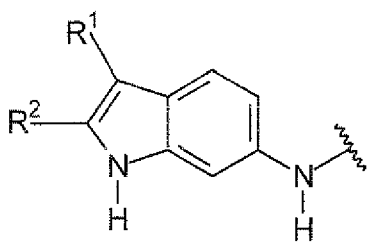

(wherein $R^1$ and $R^2$ are as defined above. Preferably $R^2$ is hydrogen and $R^1$ is

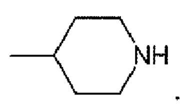

In one embodiment A has the formula (VIII)

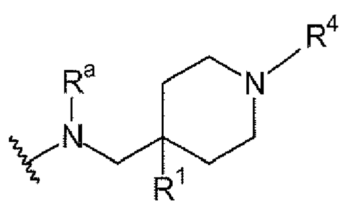

(VIII)

Preferred embodiments of (VIII) include

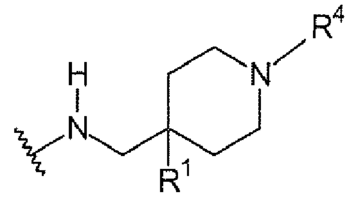

such as

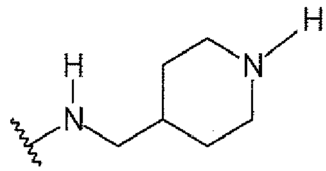

In one embodiment A has the formula (IX)

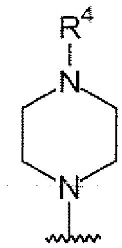

(IX)

wherein $R^4$ is as defined above (such as hydrogen).

In one embodiment A has the formula (X)

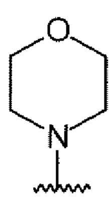

(X)

In one embodiment B has the formula (XII)

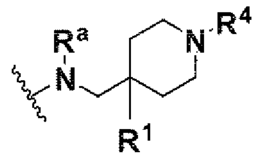

(XII)

Preferred embodiments of (XII) include,

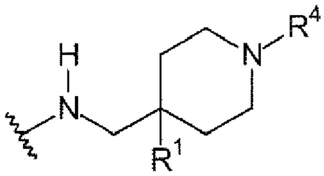

in which $R^1$ and $R^4$ are as defined above. Preferably $R^1$ is selected from the group consisting of hydrogen and halogen (such as F). Preferably $R^4$ is hydrogen or alkyl (such as methyl), more preferably hydrogen.

Specific embodiments are

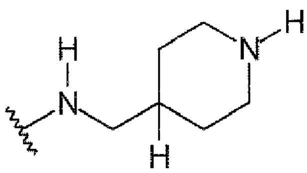

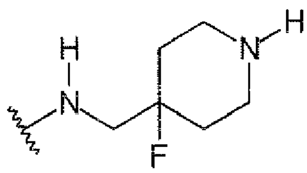

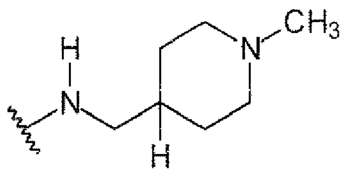

.

In one embodiment B has the formula (XIII)

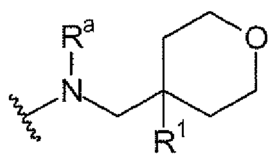

(XIII)

Preferred embodiments of (XIII) include,

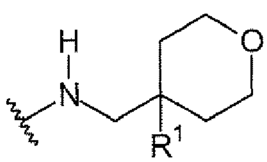

(wherein $R^1$ is as defined above). In one embodiment (XIII) is

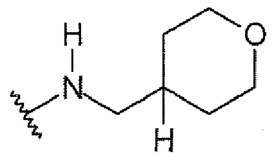

In one embodiment B has the formula (X) as defined above. In a further embodiment B has the formula (XI) as defined above.

Any combination of the above mentioned embodiments is also envisaged in the present specification.

Preferred compounds are the compounds disclosed in the example section and particularly those which are summarized in the following table.

| | |
|---|---|
| 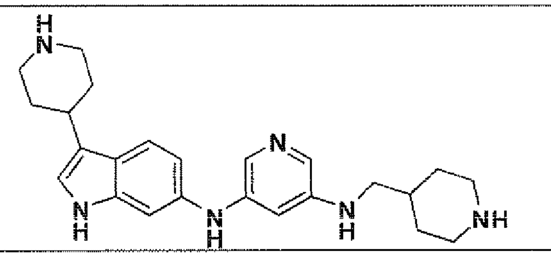 | 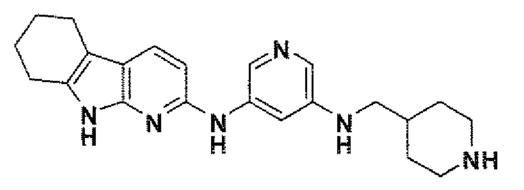 |
| 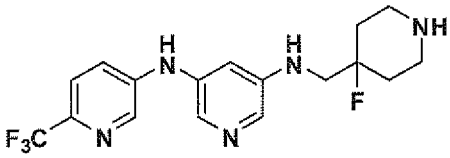 | 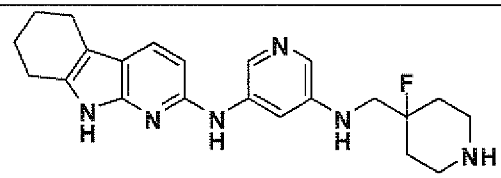 |

| | |
|---|---|
| 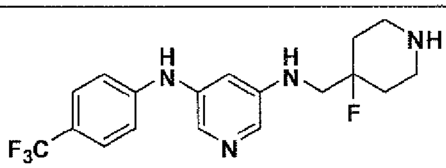 | 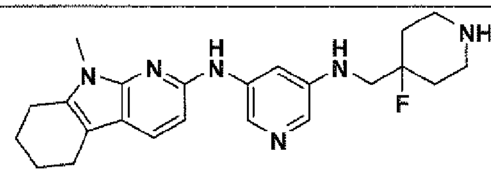 |
| 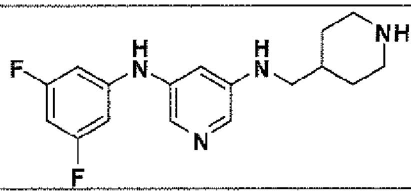 | 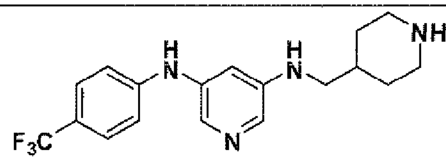 |

While it is possible for the compounds of the present invention to be administered alone, it is preferable to formulate them into a pharmaceutical composition in accordance with standard pharmaceutical practice. Thus, the invention also provides a pharmaceutical composition which comprises a therapeutically effective amount of a compound of formula (I) in admixture with a pharmaceutically acceptable excipient.

Pharmaceutically acceptable excipients are well known in the pharmaceutical art, and are described, for example, in Remington's Pharmaceutical Sciences, 1 5th Ed., Mack Publishing Co., New Jersey (1991). The pharmaceutical excipient can be selected with regard to the intended route of administration and standard pharmaceutical practice. The excipient must be acceptable in the sense of being not deleterious to the recipient thereof.

Pharmaceutically useful excipients that may be used in the formulation of the pharmaceutical composition of the present invention may comprise, for example, carriers, vehicles, diluents, solvents such as monohydric alcohols such as ethanol, isopropanol and polyhydric alcohols such as glycols and edible oils such as soybean oil, coconut oil, olive oil, safflower oil cottonseed oil, oily esters such as ethyl oleate, isopropyl myristate, binders, adjuvants, solubilizers,

thickening agents, stabilizers, disintegrants, glidants, lubricating agents, buffering agents, emulsifiers, wetting agents, suspending agents, sweetening agents, colorants, flavors, coating agents, preservatives, antioxidants, processing agents, drug delivery modifiers and enhancers such as calcium phosphate, magnesium state, talc, monosaccharides, disaccharides, starch, gelatine, cellulose, methylcellulose, sodium carboxymethyl cellulose, dextrose, hydroxypropyl-ß-cyclodextrin, polyvinylpyrrolidone, low melting waxes, and ion exchange resins.

The routes for administration (delivery) of the compounds of the invention include, but are not limited to, one or more of: oral (e. g. as a tablet, capsule, or as an ingestible solution), topical, mucosal (e. g. as a nasal spray or aerosol for inhalation), nasal, parenteral (e. g. by an injectable form), gastrointestinal, intraspinal, intraperitoneal, intramuscular, intravenous, intrauterine, intraocular, intradermal, intracranial, intratracheal, intravaginal, intracerebroventricular, intracerebral, subcutaneous, ophthalmic (including intravitreal or intracameral), transdermal, rectal, buccal, epidural and sublingual.

For example, the compounds can be administered orally in the form of tablets, capsules, ovules, elixirs, solutions or suspensions, which may contain flavoring or coloring agents, for immediate-, delayed-, modified-, sustained-, pulsed- or contralled-release applications.

The tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included. Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the agent may be combined with various sweetening or flavoring agents, coloring matter or dyes, with emulsifying and/or suspending agents and with diluents such as water, ethanol, propylene glycol and glycerin, and combinations thereof.

If the compounds of the present invention are administered parenterally, then examples of such administration include one or more of: intravenously, intraarterially, intraperitoneally, intrathecally, intraventricularly, intraurethrally, intrasternally, intracranially, intramuscularly or subcutaneously administering the compounds; and/or by using infusion techniques. For parenteral administration, the compounds are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

As indicated, the compounds of the present invention can be administered intranasally or by inhalation and are conveniently delivered in the form of a dry powder inhaler or an aerosol spray presentation from a pressurized container, pump, spray or nebulizer with the use of a suitable propellant, e. g. dichlorodifluoromethane, trichlorofluoromethane, dichlorotetrafluoroethane, a hydrofluoroalkane such as 1,1,1,2-tetrafluoroethane (NFA134AT) or 1,1,1,2,3,3,3-heptafluoropropane (HFA 227EA), carbon dioxide or other suitable gas. In the case of a pressurized aerosol, the dosage unit may be determined by providing a valve to deliver a metered amount. The pressurized container, pump, spray or nebulizer may contain a solution or suspension of the active compound, e. g. using a mixture of ethanol and the propellant as the solvent, which may additionally contain a lubricant, e. g. sorbitan trioleate. Capsules and cartridges (made, for example, from gelatin) for use in an inhaler or insufflator may be formulated to contain a powder mix of the compound and a suitable powder base such as lactose or starch.

Alternatively, the compounds of the present invention can be administered in the form of a suppository or pessary, or it may be applied topically in the form of a gel, hydrogel, lotion, solution, cream, ointment or dusting powder. The compounds of the present invention may also be dermally or transdermally administered, for example, by the use of a skin patch.

They may also be administered by the pulmonary or rectal routes. They may also be administered by the ocular route. For ophthalmic use, the compounds can be formulated as micronized suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For application topically to the skin, the compounds of the present invention can be formulated as a suitable ointment containing the active compound suspended or dissolved in, for example, a mixture with one or more of the following: mineral oil, liquid petrolatum, white petrolatum, propylene glycol, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

Typically, a physician will determine the actual dosage which will be most suitable for an individual subject. The specific dose level and frequency of dosage for any particular individual may be varied and will depend upon a

variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy.

A proposed dose of the compounds according to the present invention for administration to a human (of approximately 70 kg body weight) is 0.1 mg to 1 g, preferably 1 mg to 500 mg of the active ingredient per unit dose. The unit dose may be administered, for example, 1 to 4 times per day. The dose will depend on the route of administration. It will be appreciated that it may be necessary to make routine variations to the dosage depending on the age and weight of the patient as well as the severity of the condition to be treated. The precise dose and route of administration will ultimately be at the discretion of the attendant physician or veterinarian.

The compounds of the invention may also be used in combination with other therapeutic agents. When a compound of the invention is used in combination with a second therapeutic agent active against the same disease the dose of each compound may differ from that when the compound is used alone.

The combinations referred to above may conveniently be presented for use in the form of a pharmaceutical formulation. The individual components of such combinations may be administered either sequentially or simultaneously in separate or combined pharmaceutical formulations by any convenient route. When administration is sequential, either the compound of the invention or the second therapeutic agent may be administered first. When administration is simultaneous, the combination may be administered either in the same or different pharmaceutical composition. When combined in the same formulation it will be appreciated that the two compounds must be stable and compatible with each other and the other components of the formulation. When formulated separately they may be provided in any convenient formulation, conveniently in such manner as are known for such compounds in the art.

The pharmaceutical compositions of the invention can be produced in a manner known per se to the skilled person as described, for example, in Remington's Pharmaceutical Sciences, 15th Ed., Mack Publishing Co., New Jersey (1991).

Diseases that can be treated with the compounds of the present invention can be associated with the formation of abnormal protein structures, in particular abnormal β-sheet structures. In the context of the present invention, an abnormal protein structure is a protein structure that arises when a protein or peptide refolds from the three-dimensional structure, which it generally adopts in healthy individuals, into a different three-dimensional structure, which is associated with a pathological condition. Likewise, an abnormal β-sheet structure in the context of the present invention is a β-sheet structure that arises when a protein or peptide refolds from the three-dimensional structure, which it generally adopts in healthy individuals, into a β-sheet structure, which is associated with a pathological condition.

In particular, in one embodiment diseases that can be treated with the compounds of the present invention are diseases or conditions associated with amyloid or amyloid-like proteins.

This group of diseases and disorders include neurological disorders such as Alzheimer's disease (AD), diseases or conditions characterized by a loss of cognitive memory capacity such as, for example, mild cognitive impairment (MCI), Lewy body dementia, Down's syndrome, hereditary cerebral hemorrhage with amyloidosis (Dutch type); the Guam Parkinson-Dementia complex. Other diseases which are based on or associated with amyloid-like proteins are progressive supranuclear palsy, multiple sclerosis; Creutzfeld Jacob disease, Parkinson's disease, HIV-related dementia, ALS (amyotropic lateral sclerosis), inclusion-body myositis (IBM), Adult Onset Diabetes; senile cardiac amyloidosis; endocrine tumors, and other diseases, including amyloid-associated ocular diseases that target different tissues of the eye, such as the visual cortex, including cortical visual deficits; the anterior chamber and the optic nerve, including glaucoma; the lens, including cataract due to beta-amyloid deposition; the vitreous, including ocular amyloidosis; the retina, including primary retinal degenerations and macular degeneration, in particular age-related macular degeneration; the optic nerve, including optic nerve drusen, optic neuropathy and optic neuritis; and the cornea, including lattice dystrophy.

In a preferred embodiment the compounds of the present invention can be employed for the treatment of Alzheimer's disease, mild cognitive impairment (MCI), Lewy body dementia (LBD), amyotropic lateral sclerosis (ALS), inclusion-body myositis (IBM) and age-related macular degeneration (AMD). In a particulary preferred embodiment the compounds of the present invention can be employed for the treatment of Alzheimer's disease.

The ability of a compound to inhibit the aggregation of Aβ can, for example, be determined using fluorescence correlation spectroscopy as described in Rzepecki et al., J. Biol. Chem., 2004, 279(46), 47497-47505 or by using the thioflavin T spectrofluorescence assay.

In another embodiment the compounds of the present invention can be used for treating or alleviating the effects of ocular diseases associated with pathological abnormalities/changes in the tissues of the visual system, particularly associated with amyloid-beta-related pathological abnormalities/changes in the tissues of the visual system, such as, for example, neuronal degradation. Said pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice

dystrophy.

The compounds according to the present invention can also be provided in the form of a mixture with at least one further biologically active compound and/or a pharmaceutically acceptable carrier and/or a diluent and/or an excipient. The compound and/or the further biologically active compound are preferably present in a therapeutically effective amount.

The nature of the further biologically active compound will depend on the intended use of the mixture. The further biologically active substance or compound may exert its biological effect by the same or a similar mechanism as the compound according to the invention or by an unrelated mechanism of action or by a multiplicity of related and/or unrelated mechanisms of action.

Generally, the further biologically active compound may include neutron-transmission enhancers, psychotherapeutic drugs, acetylcholine esterase inhibitors, calcium-channel blockers, biogenic amines, benzodiazepine tranquillizers, acetylcholine synthesis, storage or release enhancers, acetylcholine postsynaptic receptor agonists, monoamine oxidase-A or -B inhibitors, N-methyl-B-aspartate glutamate receptor antagonists, non-steroidal anti-inflammatory drugs, antioxidants, and serotonergic receptor antagonists. In particular, the further biologically active compound can be selected from the group consisting of a compound used in the treatment of amyloidosis, compounds against oxidative stress, anti-apoptotic compounds, metal chelators, inhibitors of DNA repair such as pirenzepin and metabolites, 3-amino-1-propanesulfonic acid (3APS), 1,3-propanedisulfonate (1,3PDS), α-secretase activators, β- and γ-secretase inhibitors, tau proteins, neurotransmitter, β-sheet breakers, attractants for amyloid beta clearing / depleting cellular components, inhibitors of N-terminal truncated amyloid beta including pyroglutamated amyloid beta 3-42, anti-inflammatory molecules, or cholinesterase inhibitors (ChEls) such as tacrine, rivastigmine, donepezil, and/or galantamine, M1 agonists, other drugs including any amyloid or tau modifying drug and nutritive supplements, an antibody, including any functionally equivalent antibody or functional parts thereof, an Aβ antigenic peptide fragment consisting of a single or repetitive stretch of a plurality of contiguous amino acid residues from the N-terminal part of the Aβ peptide.

In a further embodiment, the mixtures according to the invention may comprise niacin or memantine together with a compound according to the present invention and, optionally, a pharmaceutically acceptable carrier and/or a diluent and/or an excipient.

In still another embodiment of the invention mixtures are provided that comprise as a further biologically active compound "atypical antipsychotics" such as, for example clozapine, ziprasidone, risperidone, aripiprazole or olanzapine for the treatment of positive and negative psychotic symptoms including hallucinations, delusions, thought disorders (manifested by marked incoherence, derailment, tangentiality), and bizarre or disorganized behavior, as well as anhedonia, flattened affect, apathy, and social withdrawal, together with a compound according to the invention and, optionally, a pharmaceutically acceptable carrier and/or a diluent and/or an excipient.

Other compounds that can be suitably used in mixtures in combination with the compound according to the present invention are, for example, described in WO 2004/058258 (see especially pages 16 and 17) including therapeutic drug targets (pages 36 to 39), alkanesulfonic acids and alkanolsulfuric acids (pages 39 to 51), cholinesterase inhibitors (pages 51 to 56), NMDA receptor antagonists (pages 56 to 58), estrogens (pages 58 to 59), non-steroidal anti-inflammatory drugs (pages 60 and 61), antioxidants (pages 61 and 62), peroxisome proliferators-activated receptor (PPAR) agonists (pages 63 to 67), cholesterol-lowering agents (pages 68 to 75), amyloid inhibitors (pages 75 to 77), amyloid formation inhibitors (pages 77 to 78), metal chelators (pages 78 and 79), anti-psychotics and anti-depressants (pages 80 to 82), nutritional supplements (pages 83 to 89) and compounds increasing the availability of biologically active substances in the brain (see pages 89 to 3) and prodrugs (pages 93 and 94), which document is incorporated herein by reference.

In one preferred embodiment the further biologically active compound is an antibody including any functionally equivalent antibody or functional parts thereof. The antibody can preferably be monoclonal, chimeric or humanized.

In a further aspect of the invention, a mixture is provided comprising in addition to the compound of the invention an antibody including functional parts thereof, or, more particularly, a monoclonal antibody including functional parts thereof, which recognizes and binds to amyloid β (Aβ), particularly to the native conformation of amyloid β, that is to amyloid oligomers and fibers, but not to not linearized amyloid species.

In particular, said antibodies are capable of inhibiting, *in vitro* and *in vivo*, the aggregation of amyloidogenic monomeric peptides, specifically β-amyloid monomeric peptides such as, for example, Aβ monomeric peptides 1-39; 1-40, 1-41, 1-42, or 1-43, but especially $A\beta_{1-42}$ monomeric peptides, into high molecular polymeric amyloid fibrils or filaments. Through the inhibition of the aggregation of amyloidogenic monomeric peptides these antibodies are capable of preventing or slowing down the formation of amyloid plaques, particularly the amyloid form (1-42), which is known to become insoluble by change of secondary conformation and to be the major part of amyloid plaques in brains of diseased animals or humans.

In another aspect of the invention, the mixture comprises antibodies which, upon co-incubation with preformed high molecular polymeric amyloid fibrils or filaments formed by the aggregation of amyloid- monomeric peptides, specifically β-amyloid monomeric peptides such as, for example, Aβ monomeric peptides 1-39; 1-40, 1-41, 1-42, or 1-43,

but especially $A\beta_{1-42}$ monomeric peptides, are capable of disaggregating said high molecular polymeric amyloid fibrils or filaments. Through the disaggregation of amyloidogenic polymeric fibrils or filaments these antibodies are capable of preventing or slowing down the formation of amyloid plaques which leads to an alleviation of the symptoms associated with the disease and a delay or reversal of its progression.

In still another aspect of the invention, the mixture comprises an antibody, but especially a monoclonal antibody or functional parts thereof, which antibody is bifunctional or bispecific in that it exhibits both an aggregation inhibition property as well as a disaggregation property as defined herein before, particularly paired with a high degree of conformational sensitivity.

In one embodiment, the mixture comprises an antibody which recognizes and binds to a conformational epitope, particularly a conformational epitope which is present in the N-terminal part of the amyloid β peptide, particularly embedded into the following core region of the N-terminal part of the amyloid β peptide:

| | | | | | | | | | | | |
|---|---|---|---|---|---|---|---|---|---|---|---|
| Val-12 | His-13 | His-14 | Gln-15 | Lys-16 | Leu-17 | Val-18 | Phe-19 | Phe-20 | Ala-21 | Glu-22 | Asp-23 |

Particularly an epitope localized in a region of the β-amyloid protein between amino acid residue 12 to 24, particularly between residues 14 to 23, more particularly between amino acid residues 14 and 20, comprising three distinct recognition and binding sites which residues are predominantly involved in the binding of the β-amyloid protein and located at position 16, 17, and at position 19 and 20, and at position 14, respectively.

In a specific embodiment the mixture of the present invention comprises, in addition to the compound of the invention, an antibody, particularly a bifunctional antibody, but especially a monoclonal antibody, particularly a bifunctional monoclonal antibody, including any functionally equivalent antibody or functional parts thereof, which antibody has the characteristic properties of an antibody produced by a hybridoma cell line selected from the group consisting of FP 12H3, FP 12H3-C2, and FP 12H3-G2 deposited on December 01, 2005 and December 09, 2005, respectively, as DSM ACC2752, DSM ACC 2750 and DSM ACC2751, respectively, ET 7E3 deposited on December 08, 2005 as DSM ACC2755, and EJ 7H3 deposited on December 08, 2005 as DSM ACC2756.

More particularly, the invention relates to an antibody including any functionally equivalent antibody or functional parts thereof, produced by a hybridoma cell line selected from the group consisting of FP 12H3, FP 12H3-C2, and FP 12H3-G2 deposited on December 01, 2005 and December 09, 2005, respectively, as DSM ACC2752, DSM ACC 2750 and DSM ACC2751, respectively, ET 7E3 deposited on December 08, 2005 as DSM ACC2755, and EJ 7H3 deposited on December 08, 2005 as DSM ACC2756.

The above antibodies are described in the published international application WO 2007/068412, which is incorporated herein by reference.

In a further aspect, the antibody which is comprised in the mixture according to the invention is a chimeric antibody or a fragment thereof, or a humanized antibody or a fragment thereof. These and further antibodies that can be suitably used within the mixtures according to the present invention are described, for example, in international application PCT/US2007/073504 filed July 13, 2007.

If the antibody is a humanized antibody, it preferably exhibits a light chain and a heavy chain as depicted in SEQ ID No. 13 and SEQ ID No. 16 of International Application No. PCT/US2007/073504 or exhibits a light chain variable region and a heavy chain variable region as depicted in SEQ ID No. 12 and SEQ ID No. 15 of International Application No. PCT/US2007/073504. These sequences are also shown in the attached sequence listing.

In still another aspect of the invention, a mixture is provided which comprises, in addition to the compound according to the invention and as described herein before, a peptide fragment from the N-terminal part of the Aβ peptide, particularly an Aβ peptide fragment consisting of a single or repetitive stretch of between 13 and 15 contiguous amino acid residues from the N-terminal part of the Aβ peptide, but particularly an Aβ peptide fragment consisting of amino acid residues selected from the group consisting of residues 1-15, 1-14, and 1-13 from the N-terminal part of the Aβ peptide, more particularly of residue 1-15, including functionally equivalent fragments thereof, but especially a Aβ peptide fragment as mentioned herein before attached to, or incorporated or reconstituted in a carrier particle/adjuvant such as, for example, a liposome. The peptide fragment can be comprised in a vaccine composition. In particular, the peptide antigen is modified by a lipophilic or hydrophobic moiety, that facilitates insertion into the lipid bilayer of the liposome carrier/immune adjuvant, particularly by a lipophilic or hydrophobic moiety which functions as an anchor for the peptide in the liposome bilayer and has a dimension that leads to the peptide being positioned and stabilized in close proximity to the liposome surface.

In a further embodiment of the invention, the lipophilic or hydrophobic moiety is a fatty acid, a triglyceride or a phospholipid, but especially a fatty acid, a triglyceride or a phospholipid. In particular, the hydrophobic moiety is palmitic acid and the liposome preparation may in addition contain an adjuvant such as, for example, lipid A, alum, calcium phosphate, interleukin-1, and/or microcapsules of polysaccharides and proteins, but particularly a detoxified lipid A,

such as monophosphoryl or diphosphoryl lipid A, or alum.

These and further compositions that can be suitably used in the mixtures of the present invention are described, for example, in the published international application WO 2007/068411.

Diagnosis of an amyloid-associated disease or condition or of a predisposition to an amyloid-associated disease or condition in a patient may be achieved by detecting the specific binding of a compound according to the invention to the amyloid protein in a sample or *in situ*, which includes bringing the sample or a specific body part or body area suspected to contain the amyloid antigen into contact with a compound of the invention which binds the amyloid protein, allowing the compound of the invention to bind to the amyloid protein to form a compound/protein complex, detecting the formation of the compound/protein complex and correlating the presence or absence of the compound/protein complex with the presence or absence of amyloid protein in the sample or specific body part or area, optionally comparing the amount of said compound/protein complex to a normal control value, wherein an increase in the amount of said aggregate compared to a normal control value may indicate that said patient is suffering from or is at risk of developing an amyloid-associated disease or condition.

Monitoring minimal residual disease in a patient following treatment with a compound or a mixture according to the invention may be achieved by detecting the specific binding of a compound according to the invention to the amyloid protein in a sample or *in situ*, which includes bringing the sample or a specific body part or body area suspected to contain the amyloid antigen into contact with a compound of the invention which binds the amyloid protein, allowing the compound to bind to the amyloid protein to form a compound/protein complex, detecting the formation of the compound/protein complex and correlating the presence or absence of the compound/protein complex with the presence or absence of amyloid protein in the sample or specific body part or area, optionally comparing the amount of said compound/protein complex to a normal control value, wherein an increase in the amount of said aggregate compared to a normal control value may indicate that said patient may still suffer from a minimal residual disease.

Predicting responsiveness of a patient to a treatment with a compound or composition or a mixture according to the invention may be achieved by detecting the specific binding of a compound according to the invention to the amyloid protein in a sample or *in situ*, which includes bringing the sample or a specific body part or body area suspected to contain the amyloid protein into contact with a compound of the invention which binds the amyloid protein, allowing the compound to bind to the amyloid protein to form a compound/protein complex, detecting the formation of the compound/protein complex and correlating the presence or absence of the compound/protein complex with the presence or absence of amyloid protein in the sample or specific body part or area, optionally comparing the amount of said compound/protein complex before and after onset of the treatment, wherein a decrease in the amount of said aggregate may indicate that said patient has a high potential of being responsive to the treatment.

In a further aspect of the present invention, the compound of formula (I) can contain a radionuclide (e.g., $^{125}I$, $^{124}I$, $^{123}I$ or $^{18}F$). These compounds can be useful for *in vivo* diagnosis or imaging of amyloid-associated diseases, preferably of Alzheimer's disease, for example in methods such as single photon emission computed tomography (SPECT imaging) or positron emission tomography (PET).

In radiopharmaceutical applications, the compound of the present invention is preferably administered in a radiopharmaceutical formulation comprising the compound of the invention. A "radiopharmaceutical formulation" is defined in the present invention as a formulation comprising compound of the present invention (such as a compound of formula (I) or a salt thereof) in a form suitable for administration to mammals such as humans. Preferably a radiopharmaceutical formulation further comprises a physiologically acceptable excipient. Administration is preferably carried out by injection of the formulation as an aqueous solution. Such a formulation may optionally contain further ingredients such as buffers; pharmaceutically acceptable solubilisers (e.g., cyclodextrins or surfactants such as Pluronic, Tween or phospholipids); pharmaceutically acceptable stabilisers or antioxidants (such as ascorbic acid, gentisic acid or para-aminobenzoic acid). The dose of the compound of the present invention will vary depending on the exact compound to be administered, the weight of the patient, and other variables as would be apparent to a physician skilled in the art. Generally, the dose would lie in the range 0.001$\mu$g/kg to 10 $\mu$g/kg, preferably 0.01 $\mu$g/kg to 1.0 $\mu$g/kg.

Biological samples that may be used in the diagnosis of an amyloid-associated disease or condition for diagnosing a predisposition to an amyloid-associated disease or condition or for monitoring minimal residual disease in a patient or for predicting responsiveness of a patient to a treatment with a compound or a composition or a mixture according to the invention and as described herein before are, for example, fluids such as serum, plasma, saliva, gastric secretions, mucus, cerebrospinal fluid, lymphatic fluid, and the like, or tissue or cell samples obtained from an organism such as neural, brain, cardiac or vascular tissue. For determining the presence or absence of the amyloid protein in a sample any immunoassay known to those of ordinary skill in the art (see Harlow and Lane, Antibodies: A Laboratory Manual (Cold Spring Harbor Laboratory, New York, 1988, 555 to 612) may be used such as, for example, assays which utilize indirect detection methods using secondary reagents for detection, ELISA's and immunoprecipitation and agglutination assays. A detailed description of these assays is, for example, given in WO96/13590 to Maertens and Stuyver, Zrein et al. (1998) and WO96/29605.

For *in situ* diagnosis, the compound or compostion or mixture according to the invention and as described

herein before may be administered to the organism to be diagnosed by methods known in the art such as, for example, intravenous, intranasal, intraperitoneal, intracerebral, intraarterial injection such that a specific binding between the compound according to the invention and the amyloid antigen may occur. The compound/protein complex may be detected through a label attached to the compound.

The immunoassays used in diagnostic applications or in applications for diagnosing a predisposition to an amyloid-associated disease or condition or for monitoring minimal residual disease in a patient or for predicting responsiveness of a patient to a treatment with a compound or composition or a mixture according to the invention and as described herein before, typically rely on labelled antigens, antibodies, or secondary reagents for detection. These proteins or reagents can be labelled with compounds generally known to those skilled in the art including enzymes, radioisotopes, and fluorescent, luminescent and chromogenic substances including colored particles, such as colloidal gold and latex beads. Of these, radioactive labelling can be used for almost all types of assays and with most variations.

Enzyme-conjugated labels are particularly useful when radioactivity must be avoided or when quick results are needed. Fluorochromes, although requiring expensive equipment for their use, provide a very sensitive method of detection. Antibodies useful in these assays include monoclonal antibodies, polyclonal antibodies, and affinity purified polyclonal antibodies.

Alternatively, the compound of the invention may be labelled indirectly by reaction with labelled substances that have an affinity for immunoglobulin, such as protein A or G or second antibodies. The antibody may be conjugated with a second substance and detected with a labelled third substance having an affinity for the second substance conjugated to the antibody. For example, the antibody may be conjugated to biotin and the antibody-biotin conjugate detected using labelled avidin or streptavidin. Similarly, the antibody may be conjugated to a hapten and the antibody-hapten conjugate detected using labelled anti-hapten antibody.

Those of ordinary skill in the art will know of these and other suitable labels which may be employed in accordance with the present invention. The binding of these labels to antibodies or fragments thereof can be accomplished using standard techniques commonly known to those of ordinary skill in the art. Typical techniques are described by Kennedy, J. H., et al., 1976 (Clin. Chim. Acta 70:1-31), and Schurs, A. H. W. M., et al. 1977 (Clin. Chim Acta 81:1-40). Coupling techniques mentioned in the latter are the glutaraldehyde method, the periodate method, the dimaleimide method, and others, all of which are incorporated by reference herein.

Current immunoassays utilize a double antibody method for detecting the presence of an analyte, wherein the antibody is labelled indirectly by reactivity with a second antibody that has been labelled with a detectable label. The second antibody is preferably one that binds to antibodies of the animal from which the monoclonal antibody is derived. In other words, if the monoclonal antibody is a mouse antibody, then the labelled, second antibody is an anti-mouse antibody. For the monoclonal antibody to be used in the assay described below, this label is preferably an antibody-coated bead, particularly a magnetic bead. For the polyclonal antibody to be employed in the immunoassay described herein, the label is preferably a detectable molecule such as a radioactive, fluorescent or an electrochemiluminescent substance.

An alternative double antibody system, often referred to as fast format systems because they are adapted to rapid determinations of the presence of an analyte, may also be employed within the scope of the present invention. The system requires high affinity between the antibody and the analyte. According to one embodiment of the present invention, the presence of the amyloid antigen is determined using a pair of antibodies, each specific for amyloid antigen. One of said pairs of antibodies is referred to herein as a "detector antibody" and the other of said pair of antibodies is referred to herein as a "capture antibody". The monoclonal antibody can be used as either a capture antibody or a detector antibody. The monoclonal antibody can also be used as both capture and detector antibody, together in a single assay. One embodiment of the present invention thus uses the double antibody sandwich method for detecting amyloid antigen in a sample of biological fluid. In this method, the analyte (amyloid antigen) is sandwiched between the detector antibody and the capture antibody, the capture antibody being irreversibly immobilized onto a solid support. The detector antibody would contain a detectable label, in order to identify the presence of the antibody-analyte sandwich and thus the presence of the analyte.

Exemplary solid phase substances include, but are not limited to, microtiter plates, test tubes of polystyrene, magnetic, plastic or glass beads and slides which are well known in the field of radioimmunoassay and enzyme immunoassay. Methods for coupling antibodies to solid phases are also well known to those skilled in the art. More recently, a number of porous material such as nylon, nitrocellulose, cellulose acetate, glass fibers and other porous polymers have been employed as solid supports.

The plaque burden in the tissue and/or body fluid (such as the retinal ganglion cell layer of an animal, particularly a mammal, but especially a human suffering from an ocular disease associated with pathological abnormalities/changes in the tissues of the visual system, particularly associated with amyloid-beta-related pathological abnormalities/changes in the tissues of the visual system) can be calculated by methods known in the art such as that disclosed in Ding, J., et al., "Targeting age-related macular degeneration with Alzheimer's disease based immunotherapies: Anti-amyloid-b antibody attenuates pathologies in an age-related macular degeneration mouse model", Vision Research (2007), doi:

10.1016/j.visres.2007.07.025.

A compound according to the present invention can also be incorporated into a test kit for detecting an amyloid protein. The test kit typically comprises a container holding one or more compounds according to the present invention and instructions for using the compound for the purpose of binding to an amyloid protein to form a compound/protein complex and detecting the formation of the compound/protein complex such that presence or absence of the compound/ protein complex correlates with the presence or absence of the amyloid protein. The term "test kit" refers in general to any diagnostic kit known in the art. More specifically, the latter term refers to a diagnostic kit as described in Zrein et al. (1998).

The inhibition of aggregation of $A\beta_{1-42}$ by the compounds of the present invention may be measured using any suitable assay known in the art. A standard *in vitro* assay for measuring the inhibition of aggregation is described.

The synthesis of compounds of the invention inhibiting the aggregation of $A\beta_{1-42}$ and their biological activity assay are described in the following examples which are not intended to be limiting in any way.

**Generic Schemes**

The compounds of the present invention inhibiting the aggregation of $A\beta_{1-42}$ are synthesized by the general methods shown in Schemes 1 to 4.

General synthetic scheme for the preparation of 3-amino-5-bromo pyridines building blocks.

Scheme 1

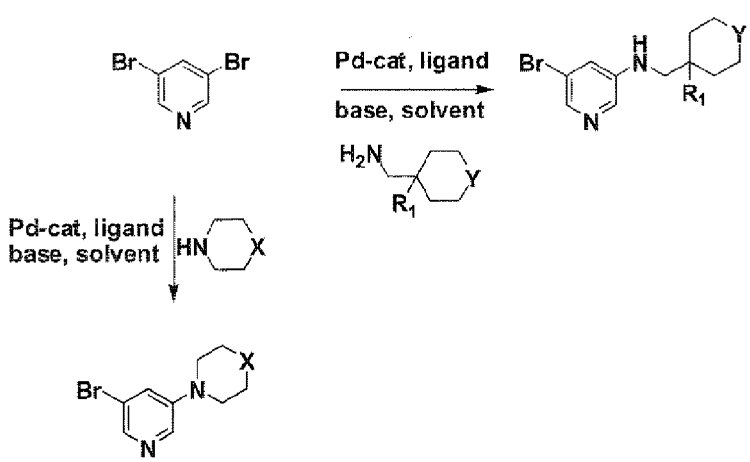


Commercially available 1,3-dibromopyridine was reacted with suitable amines or amine building blocks with (X = O, NBoc) and (Y = O, NBoc, NMe, $R_1$ = H, F) utilizing Pd-coupling chemistry with an appropriate Pd-catalyst and an appropriate ligand in a suitable solvent to afford the desired amination products after purification.

General synthetic scheme for the preparation of tricyclic 2-amino tetrahydro-pyrido[2,3-*b*]indols.

Scheme 2

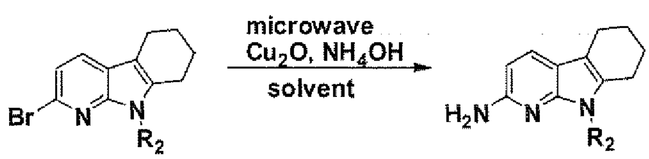


Commercially available 2,6-dibromo pyridine was treated with hydrazine hydrate in a suitable solvent to afford the mono hydrazine derivative. Condensation with cyclohexanone in a suitable solvent afforded the desired hydrazone compound. A thermal Fischer-indole synthesis afforded the tricyclic cyclization product after purification. Treatment with sodium hydride in a suitable solvent followed by methyl iodide afforded the desired N-methyl tricyclic derivative after

purification. A copper(I)-oxide mediated exchange of the bromo derivatives with ($R_2$ =H, Me) with ammonia yielded the desired tricyclic 2-amino pyrido[2,3-b]indol derivatives after purification.

General synthetic scheme for the preparation of compounds of this invention.

**Scheme 3**

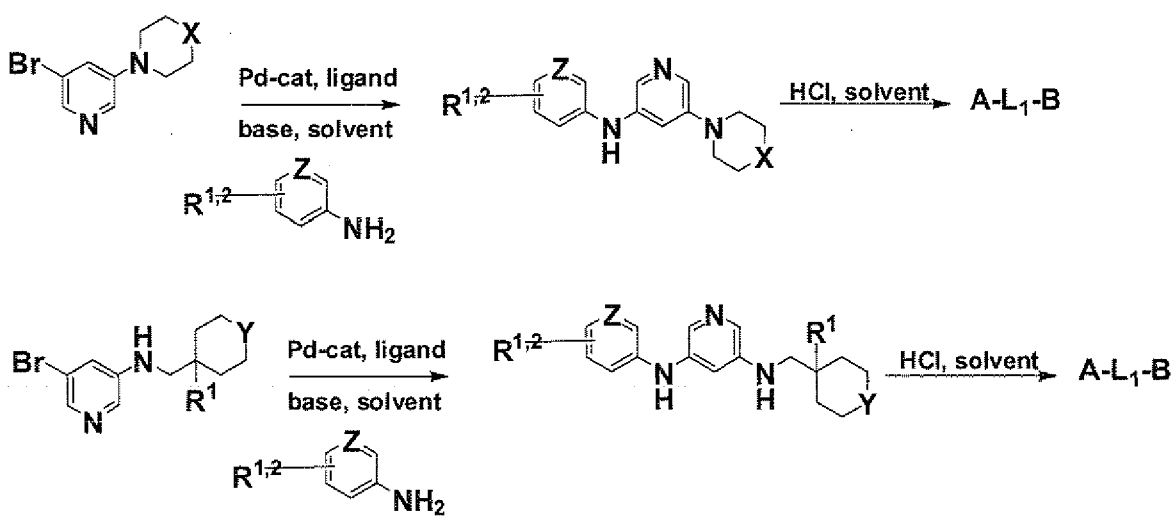


The bromo building blocks (X = O, NH) and (Y = O, NH, $R_1$ = H, F) were reacted with suitable amines or amine building blocks with (Z= CH, N) utilizing Pd-coupling chemistry with an appropriate Pd-catalyst and an appropriate ligand in a suitable solvent to afford the desired amination products after purification. Deprotection of the N-Boc compounds with hydrogen chloride in a suitable solvent afforded the final compounds.

General synthetic scheme for the preparation of compounds of this invention.

**Scheme 4**

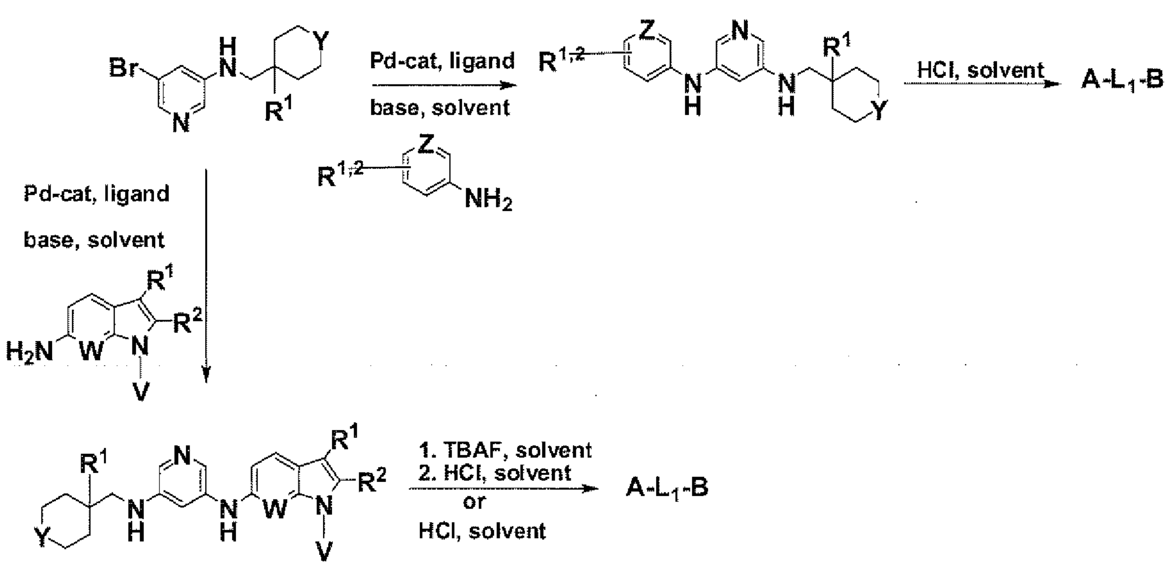


The bromo building block (Y = O, NBoc, $NCH_3$ and $R_1$ = H, F) was reacted with suitable amines or amine

building blocks with (Z= CH, N) or (W = CH, N, V = H, TIPS, $CH_3$) utilizing Pd-coupling chemistry with an appropriate Pd-catalyst and an appropriate ligand in a suitable solvent to afford the desired amination products after purification.

Deprotection of compounds with V = TIPS with tetra-n-butyl ammonium fluoride in a suitable solvent yielded the desired compounds after purification. Treatment of the TIPS-deprotected compounds with hydrogen chloride in a suitable solvent afforded the final compounds. For compounds with V = H, $CH_3$ treatment with hydrogen chloride in a suitable solvent afforded the final compounds.

## Examples

All reagents and solvents were obtained from commercial sources and used without further purification. Proton ($^1H$) spectra were recorded on a 400 MHz NMR spectrometer in deuterated solvents. Mass spectra (MS) were recorded on a Finnigan MAT TSQ 7000 spectrometer. Chromatography was performed using silica gel (Fluka: Silica gel 60, 0.063-0.2 mm) and suitable solvents as indicated in specific examples. Flash purification was conducted with a Biotage Isolera One flash purification system using HP-Sil SNAP cartridges (Biotage) and the solvent gradient indicated in specific examples. Thin layer chromatography (TLC) was carried out on silica gel plates with UV detection. Preparative thin layer chromatography (Prep-TLC) was conducted with 0.5 mm or 1 mm silica gel plates (Analtech: Uniplate, $F_{254}$) and the solvents indicated in specific examples.

## Preparative Example 1

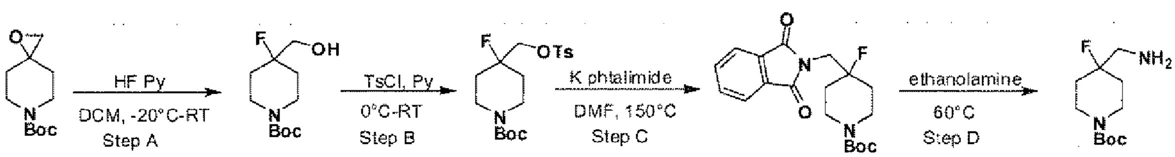


### Step A

A solution of the commercially available epoxide (2 g, 9.38 mmol) in 10 mL dichloromethane was added dropwise to a cooled mixture (-10 °C) of 0.6 mL of HF Py hydrogen fluoride in pyridine (-70%) and 10 mL of dichloromethane cooled at -10°C. Then the mixture was stirred at room temperature for 4 hours, was then diluted with dichloromethane and was washed with a saturated aqueous solution of sodium carbonate. The organic phase was separated, dried over $Na_2SO_4$, filtered and the solvents were removed to give a residue. The residue was purified by chromatography on silica using ethyl acetate/heptanes (20 to 50 %) to afford the title compound as a yellowish oil (1.44 g, 66 %).

$^1H$-NMR (400 MHz, $CDCl_3$): δ = 1.44 (s, 9H), 1.47-1.63 (m, 2H), 1.85 (m, 2H), 3.08 (m, 2H), 3.56 (s, 1H), 3.61 (s, 1H), 3.92 (brs, 2H). MS ESI: m/z = 233.98 ($M^+$)

### Step B

The product from Step A (1.44 g, 6.17 mmol) was dissolved in 6 ml of pyridine and the solution was cooled to 0°C. p-Toluenesulfonyl chloride (1.29 g, 6.79 mmol) was added and the mixture was stirred at room temperature for 4 hours. The reaction mixture was diluted with dichloromethane (250 mL) and washed with water (50 mL). The organic phase was dried over $Na_2SO_4$, filtered and the solvent was removed. The residue was purified using a Biotage flash chromatography system (ethyl acetate/n-heptane: 20 to 50%) to give a yellowish oil (2.2 g, 92%).

$^1H$-NMR (400 MHz, $CDCl_3$): δ = 7.79 (d, 2H), 7.36 (d, 2H), 3.96 (brs, 2H), 3.03 (m, 2H), 2.46 (s, 3H), 1.80 (m, 2H), 1.63-1.49 (m, 4H), 1.47 (s, 9H)

### Step C

The product from Step B (2.2 g, 5.68 mmol) was dissolved in 22 mL of dimethylformamide. Potassium phthalimide (1.052 g, 5.68 mmol) was added and the mixture was heated at 150°C for 12 hours. After cooling at room temperature, water (100 mL) was added and the mixture was extracted with ethyl acetate (3 X 250 mL). The organic phase was dried over $Na_2SO_4$, filtered and the solvent was removed to give 2.45 g of a white solid.

MS ESI: m/z = 362.95 ($M^+$)

Step D

The crude product from Step C (2.45 g) was suspended in 6 mL of ethanolamine and heated at 60˚C for 2.5h. After cooling at room temperature, water (50 mL) was added and the mixture was extracted with ethyl acetate (3 X 250 mL). The organic phase was dried over $Na_2SO_4$, filtered and the solvent was removed. The residue was purified using a Biotage flash chromatography system (methanol/dichloromethane: 20-50%) to give a yellowish oil (0.670 g, 46% for 3 steps).

$^1$H-NMR (400 MHz, $CDCl_3$): δ = 3.94 (brs, 2H), 3.06 (m, 2H), 1.84 (m, 2H), 1.56 (m, 4H), 1.44 (s, 9H)
MS (ESI): m/z = 177.04 (M-*t*-Bu+H)$^+$, 217.96 (M-Me+H)$^+$, 233.01 (MH)$^+$

**Preparative Example 2**

Step A

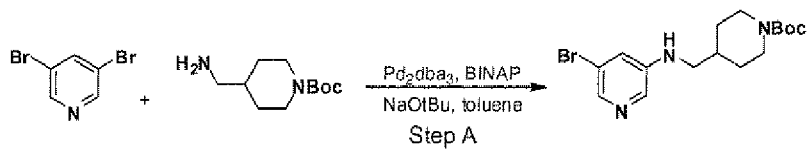


Tris(dibenzylideneacetone)dipalladium (0.193 g, 0.21 mmol), 2,2-bis-(diphenylphosphino)-1,1-naphthalene (0.26 g, 0.42 mmol), sodium *tert*-butylate (0.51 g, 5.27 mmol), 3,5-dibromopyridine (0.5 g, 2.11 mmol) and *tert*-butyl-4-(aminomethyl)piperidine-1-carboxylate (0.41 g, 1.9 mmol) were mixed into a dry Schlenk flask. The flask was evacuated under vacuum and then backfilled with argon. Then, through the septum 40 mL of toluene were added and the mixture was heated at 100 ˚C for 1 h. The reaction mixture was diluted with ethyl acetate (300 mL) and washed with water (50 mL). The organic phase was separated, dried over $Na_2SO_4$, filtered and the solvents were removed. The residue was purified by chromatography on silica using the Biotage flash chromatography system (ethyl acetate/n-heptane (4/1)) to afford the coupling product as a yellowish solid (0.5 g, 64 %).

$^1$H-NMR (400 MHz, $CDCl_3$): δ = 1.22-1.19 (m, 2H), 1.47 (s, 9H), 1.76-1.71 (m, 3H), 2.70 (dd, 2H), 3.01 (m, 2H), 4.14 (brs, 2H), 6.98 (dd, 1H), 7.91 (d, 1H), 7.97 (d, 1H)
MS (ESI): m/z = 369.91 (M-H)$^+$, 371.89 (MH)$^+$.

**Preparative Examples 3 to 10**

Following a procedure similar to that described in Preparative Example 2, except using the amines indicated in the table below, the following compounds were prepared.

| **Amine** | **Product**<br>**Preparative Example** | **1. Yield**<br>**2. $^1$H-NMR ($CDCl_3$)**<br>**3. MH$^+$** |
|---|---|---|
| 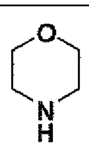 | 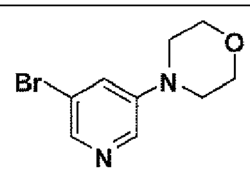<br>3 | 1. 40 %<br>2. δ = 8.2 (d, 1H), 8.14 (s, 1H), 7.27 (m, 1H), 3.85 (m, 4H), 3.19 (m, 4H)<br>3. 244.80 |

(continued)

| Amine | Product<br>Preparative Example | 1. Yield<br>2. $^{1}$H-NMR ($CDCl_3$)<br>3. $MH^+$ |
|---|---|---|
| 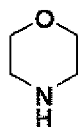 | 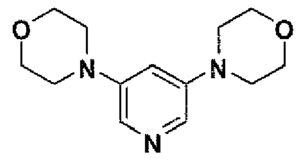<br>**4** | 1. 70 % |
| 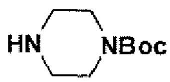 | 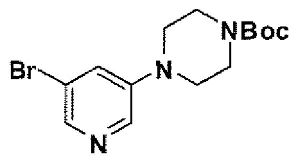<br>**5** | 1. 39 %<br>2. δ = 8.19(s, 1H), 8.12 (s, 1H), 7.28 (s, 1H), 3.57 (m, 4H), 3.16 (m, 4H), 1.47 (s, 9H) |
| 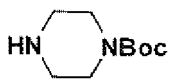 | 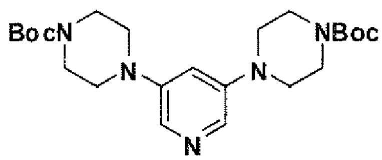<br>**6** | 1. 16 %<br>2. δ = 7.82 (s, 2H), 6.65 (s, 1H), 3.55 (m, 8H), 3.12 (m, 8H), 1.45 (s, 18H)<br>3. 448.75 |
| 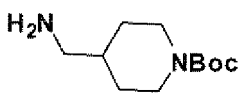 | 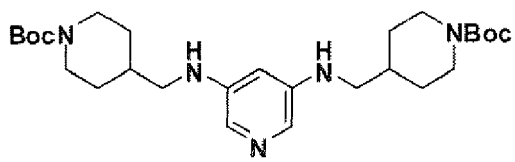<br>**7** | 1. 62 %<br>2. δ = 7.45 (s, 2H), 6.18 (s, 1H), 4.12 (brs, 4H), 3.00 (d, 4H), 2.69 (m, 4H), 1.41 (m, 6H), 1.45 (s, 18H), 1.22-1.12 (m, 4H) |
| 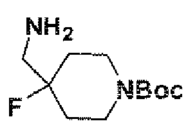 | 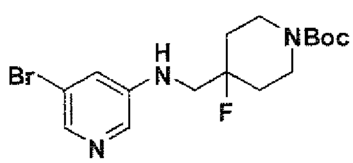<br>**8** | 1. 44 %<br>2. δ = 7.96 (d, 2H), 7.04 (s, 1H), 3.96 (brs, 2H), 3.27 (d, 1H), 3.22 (d, 1H), 3.06 (m, 2H), 1.89 (m, 2H), 1.61 (m, 1H), 1.53 (m, 1H), 1.4 (s, 9H)<br>3. 389.88 |
| 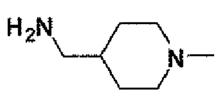 | 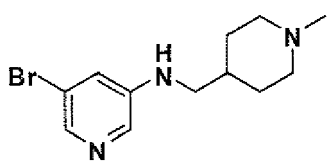<br>**9** | 1. 22 %<br>2. δ = 7.96 (d, 2H), 6.96 (s, 1H), 2.99 (m, 2H), 2.87 (m, 2H), 2.27 (s, 3H), 1.93 (t, 2H), 1.75 (d, 2H), 1.56 (m, 1H), 1.34 (m, 2H)<br>3. 85.86 |

(continued)

| Amine | Product<br>Preparative Example | 1. Yield<br>2. $^{1}$H-NMR ($CDCl_3$)<br>3. $MH^+$ |
|---|---|---|
| 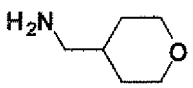 | 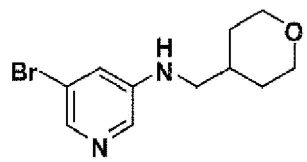<br>10 | 1. 64 %<br>2. δ = 7.96 (s, 1H), 7.91 (s, 1H), 6.98 (s, 1H), 4.0 (dd, 2H), 3.92 (brs, 1H), 3.39 (t, 2H), 3.01 (t, 2H), 1.85-1.80 (m, 1H), 1.68 (d, 2H), 1.41-1.32 (m, 2H) |

<u>Preparative Example 11</u>

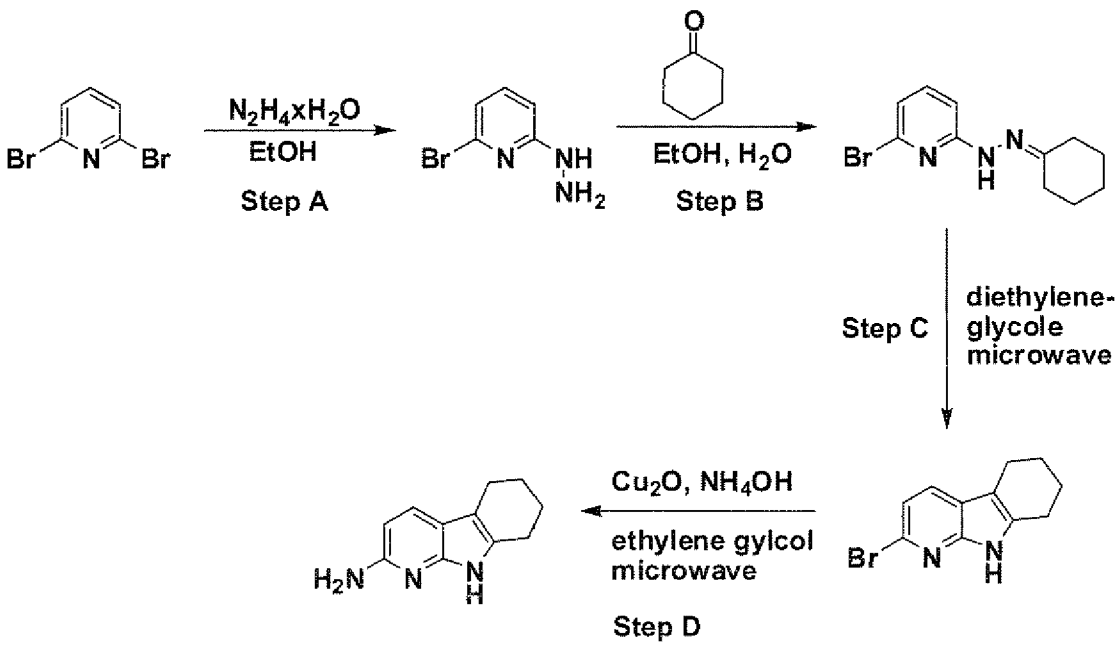


<u>Step A</u>

Commercially available 2,6-dibromopyridine (4.12 g, 16.6 mmol) was suspended in ethanol (40 mL) and hydrazine hydrate (10 mL, 97.6 mmol) in water (~50-60 %) was added. The mixture was heated in a sand-bath at ~115 ˚C for 18 h. The solvent was removed and the residue was purified by chromatography on silica using ethyl acetate/n-heptane (60/40) to afford the title compound as an off-white solid (3.05 g, 93 %).

$^{1}$H-NMR (400 MHz, $CDCl_3$): δ = 7.33 (t, 1H), 6.83 (d, 1H), 6.67 (d, 1H), 6.00 (br-s, 1H), 3.00-3.33 (br-s, 2H)

<u>Step B</u>

The title compound from Step A above (0.84 g, 4.49 mmol) was dissolved in ethanol (16 mL) and water (4 mL). After the addition of cyclohexanone (0.54 mL, 5.1 mmol), the mixture was stirred at room temperature for 1 h. The precipitate was collected by filtration, washed with ethanol (5 mL) and air-dried to afford the title compound as a white solid (0.88 g, 73%).

$^{1}$H-NMR (400 MHz, DMSO-$d_6$): δ = 9.83 (s, 1H), 7.42 (t, 1H), 7.00 (d, 1H), 6.80 (d, 1H), 2.39-2.41 (m, 2H), 2.20-2.23 (m, 2H), 1.50-1.64 (m, 6H)

Step C

The title compound from Step B above (0.2 g, 0.75 mmol) was suspended in diethylene glycol (2 mL) and heated at 250 ˚C for 30 minutes using a Biotage Initiator microwave. The mixture was diluted with ethyl acetate (40 mL) and water (15 mL). The organic phase was separated, washed with brine (10 mL), dried over $Na_2SO_4$, filtered and the solvents were removed. The residue was purified employing a Biotage Isolera One system using an ethyl acetate/n-heptane (5/95 -> 30/70) gradient to afford the title compound as an off-white solid (0.096 g, 51 %).

$^1$H-NMR (400 MHz, DMSO-$d_6$): δ = 11.40 (s, 1H), 7.63 (d, 1H), 7.05 (d, 1H), 2.68 (t, 2H), 2.57 (t, 2H), 1,73-1,85 (m, 4H)

Step D

The title compound from Step C above (0.06 g, 0.24 mmol) was suspended in ethylene glycol (2 mL) and 30 % ammonium hydroxide solution (3 mL). After the addition of copper(I)-oxide (0.005 g, 0.035 mmol), the mixture was heated at 150 ˚C for 45 minutes using a Biotage Initiator microwave. The reaction mixture was diluted with ethyl acetate (30 mL) and a mixture of water/ammonium hydroxide (10 mL, 1/1). The organic phase was separated, dried over $Na_2SO_4$, filtered and the solvents were evaporated. The residue was purified by PREP-TLC using dichloromethane/methanol (95/5) to afford the title compound as a brown solid (0.022 g, 50%).

$^1$H-NMR (400 MHz, DMSO-$d_6$): δ = 10.30 (s, 1H), 7.34 (d, 1H), 6.12 (d, 1H), 5.26 (s, 2H), 2.55 (t, 2H), 2.46 (t, 2H), 1.67-1.78 (m, 4H)

**Preparative Example 12**

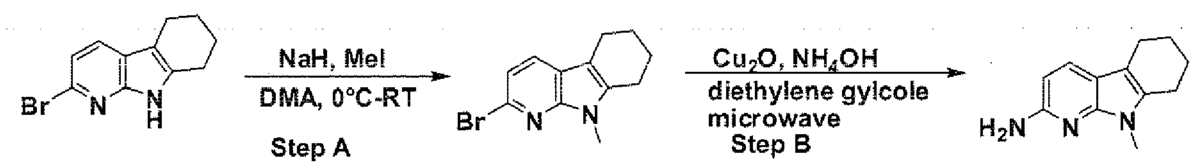


Step A

To a suspension of sodium hydride (0.02 g, 1.765 mmol) in 2 mL of N,N'-dimethylacetamide cooled at 0˚C, a solution of the title compound from Preparative Example 11 Step C (0.16 g, 0.637 mmol) in 6 mL of N,N'-dimethylacetamide was added dropwise. After the addition was completed the mixture was stirred at room temperature for one hour. Then methyl iodide (0.16 mL, 2.55 mmol) was added and the mixture was stirred at room temperature for 12 hours. The mixture was diluted with ethyl acetate (250 mL) and water (50 mL). The organic phase was separated, dried over $Na_2SO_4$, filtered and the solvents were removed. The residue obtained after solvent removal was purified using a Biotage flash chromatography system (ethyl acetate/heptanes: 10->20%) to give a white compound (0.11 g, 66 %).

$^1$H-NMR (400 MHz, $CDCl_3$) δ = 7.55 (d, 1H), 7.11 (d, 1H), 3.68 (s, 3H), 2.68 (m, 4H), 1.95 (m, 2H), 1.85 (m, 2H) MS (BSI): m/z = 264.82 (M-H)$^+$, 266.80 (MH)$^+$

Step B

The title compound from Step A above (0.11 g, 0.422 mmol) was suspended in ethylene glycol (4 mL) and 30 % ammonium hydroxide solution (6 mL). After the addition of copper(I)-oxide (0.008 g), the mixture was heated at 150 ˚C using a Biotage Initiator microwave for 1.5 hours. The reaction mixture was diluted with ethyl acetate (200 mL) and water (20 ml). The organic phase was separated, dried over $Na_2SO_4$, filtered and the solvents were evaporated. The residue was purified using a Biotage flash chromatography system (methanol/dichloromethane: 1 -> 5 %) to give a white compound (0.06 g, 70 %).

$^1$H-NMR (400 MHz, $CDCl_3$): δ = 7.51 (d, 1H), 6.28 (d, 1H), 4.27 (brs, 2H), 3.59 (s, 3H), 2.64 (m, 4H), 1.93 (m, 2H), 1.84 (m, 2H)
MS (ESI): m/z = 201.93 (M)$^+$

**Preparative Example 13**

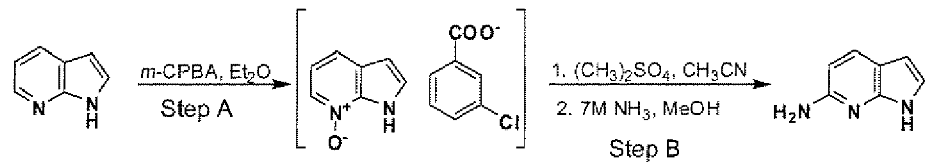


Step A

To a solution of 7-azaindole (5 g, 42.3 mmol) in diethyl ether (350 mL) was added m-CPBA (11 g, 63.4 mmol) portionwise at room temperature. The reaction mixture was stirred at room temperature for 5 hours. The precipitated product was filtered off and washed with more diethylether. The solid was collected and dissolved in boiling water and acetone mixture (50 mL: 10mL) and cooled to 5 ˚C slowly. The crystallized product was filtered and air dried to give the title compound (11.7 g, 96 %).

$^1$H-NMR (400 MHz, $CDCl_3$): δ = 12.4. (s, 1H), 8.14 (d, 1H), 7.66 (d, 1H), 7.46 (d, 1H), 7.07 (dd, 1H), 6.59 (d, 1H)

Step B

To a suspension of the compound from Step A above (2 g, 6.92 mmol) in dry acetonitrile (15 mL) was added dimethylsulfate (0.885 g, 6.92 mmol). The reaction mixture was heated at 70 ˚C for 8 hours. Then the clear solution was cooled to room temperature. The solution was distributed in three sealed tubes and cooled to 0 ˚C under an argon atmosphere. Then a 7 M solution of ammonia in methanol (5 mL) was introduced in each tube. The tubes were sealed. The sealed tubes were heated at 50 to 60 ˚C for 48 h. The solvent was removed and the residue was dissolved in ethyl acetate (200 mL) and the organic phase was washed with dilute $Na_2CO_3$ solution, water, and brine. The organic phase was dried over $Na_2SO_4$. The solvent was evaporated and crude product was purified on silica gel (ethyl acetate) to give the title compound (0.5 g, 54 %).

$^1$H-NMR (400 MHz, $CDCl_3$): δ = 7.71 (d, 9H), 6.99 (dd, 1H), 6.38 (d, J = 8.4Hz, 1H), 6.35 (dd, 1H), 4.33 (m, 2H)

**Preparative Example 14**

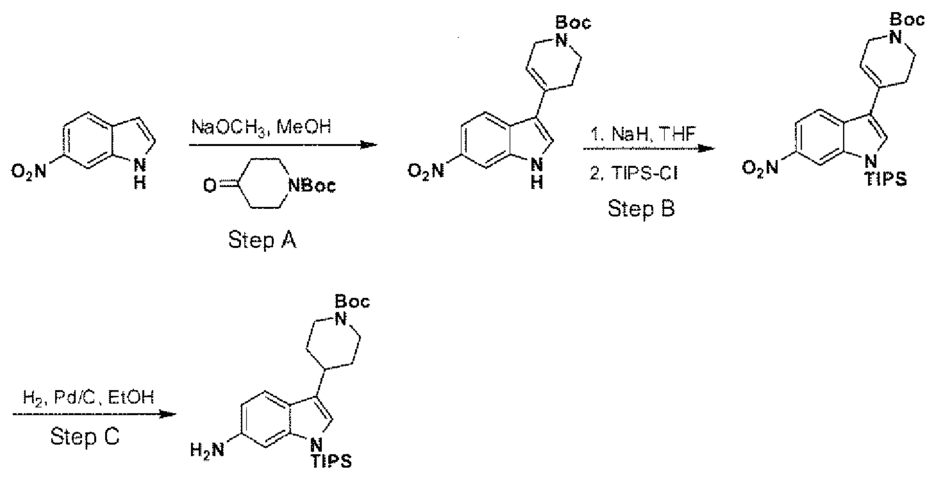

Step A

To a solution of the commercially available 6-nitroindole (2 g, 12.33 mmol) in MeOH (30 ml) was added N-Boc-4-piperidone (2.95 g, 14.80 mmol) then potassium hydroxide (2.076 g, 37.0 mmol). The resulting mixture was warmed to 75 ˚C for 3 hours. Then the reaction mixture was cooled to room temperature and the reaction mixture was concentrated to dryness. The residue was purified by flash chromatography in dichloromethane/methanol (97/3 ->95/5) to afford the title compound as a yellow solid (3.24 g, 76 %).

$^1$H-NMR (400 MHz, $DMSO_6$): δ = 11.8 (br-s, 1H), 8.31 (s, 1H), 7.98 (d, 1H), 7.84-7.90 (m, 2H), 6.16-6.19 (m, 1H), 4.00-4.04 (m, 2H), 3.54 (t, 2H), 2.48-2.51 (m, 2H), 1.39 (s, 9H)

Step B

To a solution of the title compound from Step A above (0.5 g, 1.456 mmol) in dry tetrahydrofuran (10 mL) was added at 0 ˚C sodium hydride 60% (0.056 g, 1.456 mmol) in portions. The reaction mixture was stirred at room temperature for 20 minutes then triisopropylsilyl chloride (0.312 ml, 1.456 mmol) was added. The resulting mixture was stirred at room temperature for 12 hours. The reaction mixture was concentrated to dryness. The residue was diluted with ethyl acetate. An extraction was performed with a saturated solution of sodium bicarbonate and brine. The organic layers were collected, dried over $Na_2SO_4$, filtered and concentrated to dryness. The crude product was purified by flash chromatography in ethyl acetate/n-heptane (15/85 to 40/60) to afford to the title compound as a yellow solid (0.54 g, 74%).

MS ESI: m/z = 500.05 $(MH)^+$

Step C

To a suspension of palladium 10% on carbon (0.006 g, 0.054 mmol) in ethanol (15 mL) under argon was added the title compound from Step B above (0.54 g, 1.081 mmol). The resulting mixture was placed under a hydrogen atmosphere and was stirred at room temperature for 12 hours. Then the reaction mixture was filtered over celite. The solution was concentrated to dryness. The crude product was purified by flash chromatography in an ethyl acetate/n-heptane mixture to afford the title compound as a white foam (0.35 g, 69 %).

$^1$H-NMR (400MHz, $CDCl_3$): δ=7.38 (d, 1H), 6.82 (s, 1H), 6.78 (s, 1H), 6.59 (d, 1H), 4.23 (m, 2H); 3.59 (m, 2H); 2.80-2.98 (m, 3H); 2.03 (d, 2H), 1.55-1.75 (m, 5H), 1.51 (s, 9H), 1.15(d, 18H) MS ESI: m/z = 472.23 $(MH)^+$

**Preparative Example 15**

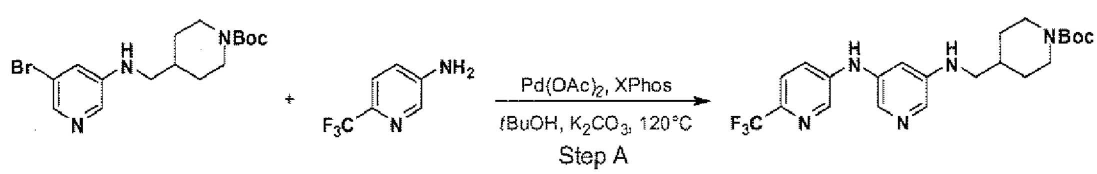


Step A

Tert-butanol (6 mL) was degassed by sonication for 1 min while a stream of argon was passed through the solution. To the degassed solvent was added palladium(II)acetate (0.006g, 0.027 mmol) and 2-dicyclohexylphosphino-2',4',6'-triisopropylbiphenyl (XPhos, 0.038 g, 0.081 mmol). This mixture was heated at ~100 ˚C in a sand-bath for 1 min to generate the catalyst. To the faint red catalyst solution were then added the title compound from Preparative Example 2 (0.1 g, 0.27 mmol) and 6-(trifluoromethyl)pyridine-3-amine (0.052 g, 0.324 mmol, 1.2 eq). After the addition of potassium carbonate (0.09 g, 0.675 mmol), the mixture was heated in a sand-bath at ~120 ˚C for 3 hours. The mixture was diluted with ethyl acetate (50 mL) and water (10 mL). The organic phase was separated, dried over $Na_2SO_4$, filtered and the solvents were removed. The residue was purified by chromatography on silica using a Biotage flash chromatography system (ethyl acetate/heptane: 50% -> 80%) to afford the title compound as a yellowish oil (0.08 g, 66 %).

$^1$H-NMR (400 MHz, $CDCl_3$): δ = 8.42 (d, 1H), 7.84 (d, 1H), 7.76 (d, 1H), 7.54 (d, 1H), 7.44 (dd, 1H), 6.69 (brs, 1H), 4.13 (brs, 2H), 3.02 (d, 2H), 2.73-2.67 (m, 2H), 1.77-1.74 (m, 3H), 1.46 (s, 9H), 1.25-1.17 (m, 2H)
MS (ESI): m/z = 452.00 $(MH)^+$

**Preparative Example 16 to 28**

Following a procedure similar to that described in Preparative Example 15, except using the bromo-derivatives and amines indicated in the table below, the following compounds were prepared.

| **Bromo-derivative** | **Amine** | **Product Preparative Example** | **1. Yield 2. $^1$H-NMR ($CDCl_3$) 3. MH$^+$** |
|---|---|---|---|
| 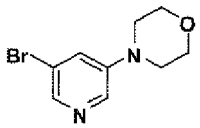 | 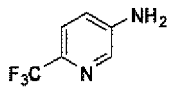 | 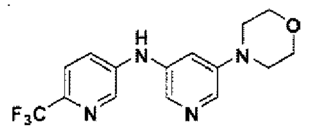 16 | 1.48%<br>3. 324.87 (M) |
| 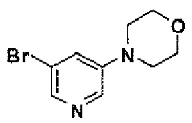 | 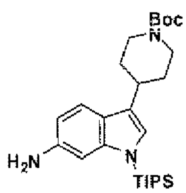 | 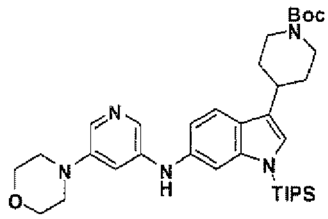 17 | 1. 85 %<br>2. δ = 7.83 (s, 1H), 7.77 (s, 1H), 7.53 (d, 1H), 7.23 (s, 1H), 6.92 (m, 2H), 6.79 (s, 1H), 4.25 (brs, 2H), 3.81 (m, 4H), 3.11 (m, 4H), 2.93-2.88 (m, 3H), 2.03 (d, 2H), 1.61 (m, 2H), 1.48 (s, 9H), 1,12 (d, 21H)<br>3.634.19 |
| 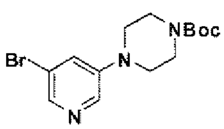 | 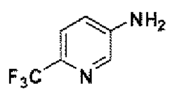 | 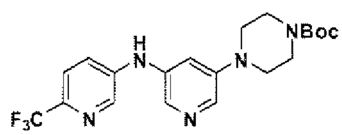 18 | 1. 79 %<br>2. δ = 8.38 (d, 1H), 7.97 (d, 1H), 7.50 (d, 1H), 7.40 (dd, 1H), 7.13 (s, 1H), 6.98 (s, 1H), 3.56 (m, 4H), 3.14 (m, 4H), 1.45 (s, 9H) |
| 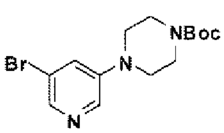 | 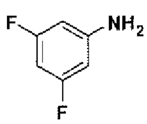 | 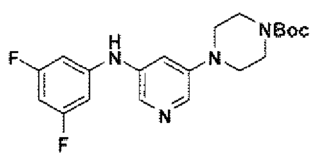 19 | 1. 70 % |
| 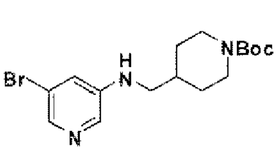 | 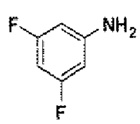 | 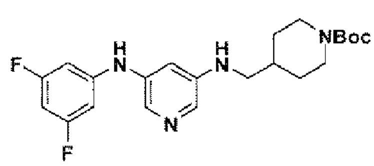 20 | 1. 58 %<br>2. δ= 7.77 (d, 1H), 7.70 (d, 1H), 6.86 (s, 1H), 6.53-6.52 (m, 2H), 6.35 (m, 1H), 4.14 (brs, 2H), 3.01 (d, 2H), 2.70 (m, 2H), 1.75(m, 3H), 1.45 (s, 9H), 1.18 (m, 2H) |

(continued)

| Bromo-derivative | Amine | Product<br>Preparative Example | 1. Yield<br>2. $^1$H-NMR ($CDCl_3$)<br>3. $MH^+$ |
|---|---|---|---|
| 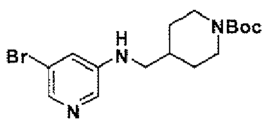 | 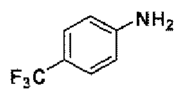 | 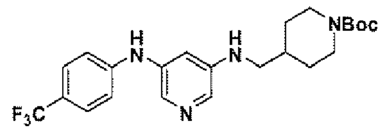<br>**21** | 1. 70 %<br>2. δ = 7.82 (d, 1H), 7.70 (d, 1H), 7.50 (d, 2H), 7.00 (d, 2H), 6.73 (s, 1H), 4.14 (brs, 2H), 3.01 (d, 2H), 2.69 (m, 2H), 1.75 (m, 3H), 1.45 (s, 9H), 1.18 (m, 2H) |
| 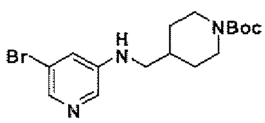 | 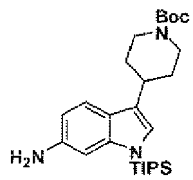 | 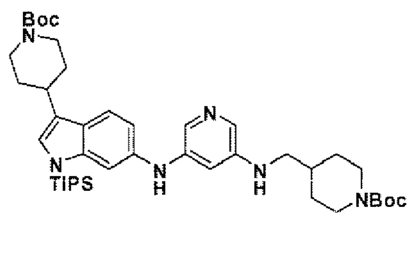<br>**22** | 1.62%<br>3. 761.40 |
| 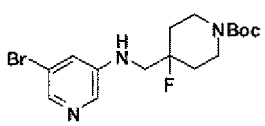 | 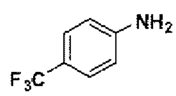 | 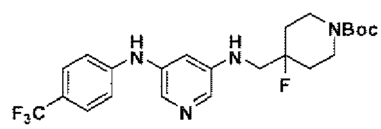<br>**23** | 1. 66 %<br>2. δ = 7.75 (d, 1H), 7.44 (d, 2H), 7.05 (d, 2H), 6.82-6.76 (m, 2H), 3.94 (brs, 2H), 3.22 (d, 2H), 3.05 (brs, 2H), 1.93-1.88 (m, 2H), 1.64-1.51 (m, 2H), 1.44 (s, 9H). |
| 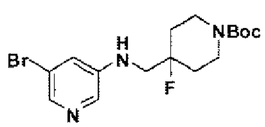 | 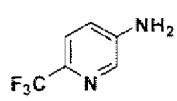 | 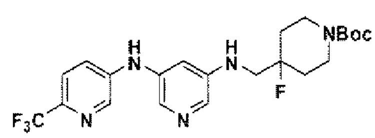<br>**24** | 1. 46 %<br>2. δ = 8.38 (d, 1H), 7.80 (dd, 2H), 7.52 (d, 1H), 7.41 (dd, 1H), 6.74 (s, 1H), 3.96 (brs, 2H), 3.25 (dd, 2H), 3.07 (m, 2H), 1.96-1.91 (m, 2H), 1.66-1.50 (m, 2H), 1.45 (s,9H).<br>3. 470.02 |

(continued)

| Bromo-derivative | Amine | Product<br>Preparative Example | 1. Yield<br>2. $^1$H-NMR ($CDCl_3$)<br>3. $MH^+$ |
|---|---|---|---|
| 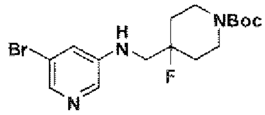 | 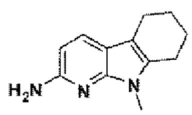 | 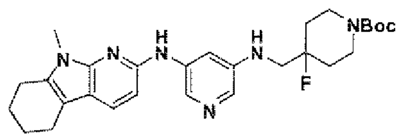<br>**25** | 1. 66 %<br>2. δ = 8.05 (s, 1H), 7.65-7.60 (m, 3H), 6.54 (d, 1H), 4.12 (brs, 2H), 3.65 (s, 3H), 3.32 (d, 2H), 3.09 (m, 2H), 2.70 (m, 2H), 2.65 (m, 2H), 1.99-1.94 (m, 4H), 1.87-1.83 (m, 2H), 1.69-1.52 (m, 2H), 1.45 (s, 9H) |
| 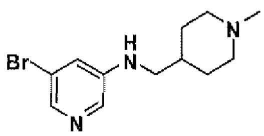 | 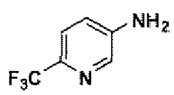 | 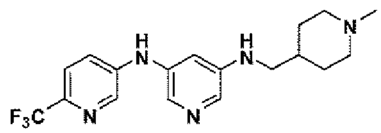<br>**26** | 1. 49 %<br>2. δ = 8.38 (s, 1H), 7.77 (d, 2H), 7.51 (d, 1H), 7.41 (s, 1H), 6.64 (s, 1H), 2.98 (m, 2H), 2.88 (d, 2H), 2.27 (s, 3H), 1.93 (t, 2H), 1.76 (d, 2H), 1.56 (brs, 1H), 1.37 (m, 2H)<br>3. 365.97 |
| 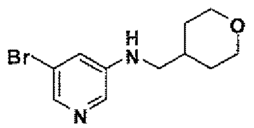 | 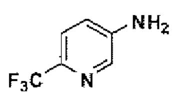 | 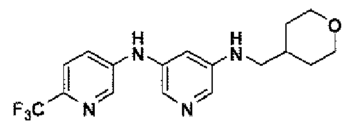<br>**27** | 1. 60 %<br>2. δ = 8.41 (d, 1H), 7.81 (d, 2H), 7.54 (d, 1H), 7.42 (d, 1H), 6.67 (s, 1H), 4.01 (dd, 2H), 3.4(t, 2H), 3.03 (t, 2H), 1.85 (m, 1H), 1.71 (d, 2H), 1.36 (m, 2H)<br>3. 352.92 $[M]^+$ |
| 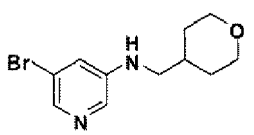 | 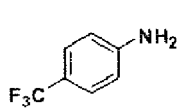 | 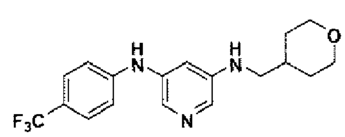<br>**28** | 1. 83 %<br>2. δ = 7.81 (s, 1H), 7.51 (d, 2H), 7.27 (s, 1H), 7.07 (d, 2H), 6.67 (s, 1H), 4.01 (dd, 2H), 3.39 (t, 2H), 3.03 (t, 2H), 1.85 (m, 1H), 1.66 (d, 2H), 1.43-1.35 (m, 2H) |

**Preparative Example 29**

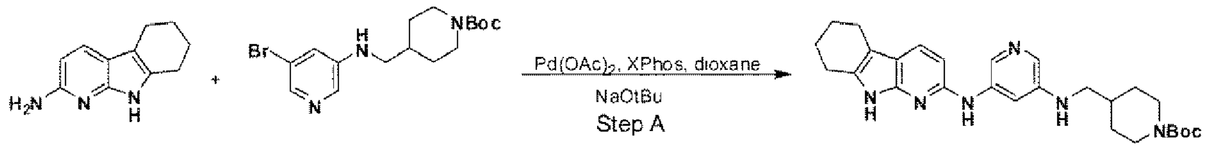


Step A

Dioxane (2 mL) was degassed by sonication for 1 min while a stream of argon was passed through the solution. To the degassed dioxane (1 mL) was added palladium(II)acetate (0.005 g, 0.023 mmol) and 2-dicyclohexylphosphino-2',4',6'-triisopropylbiphenyl (XPhos, 0.034 g, 0.07 mmol). This mixture was heated at ~100 ˚C in a sand-bath for 1 min to generate the catalyst. To the faint red catalyst solution was then added a solution of the title compounds from Preparative Example 11 (0.044 g, 0.235 mmol) and Preparative Example 2 (0.087 g, 0.235 mmol) in degassed dioxane (4 mL). After the addition of sodium tert.-butoxide (0.057 g, 0.587 mmol), the mixture was heated in a sand-bath at ~120 ˚C for 3 hours. The mixture was diluted with ethyl acetate (50 mL), water (20 mL) and brine (10 mL). The organic phase was separated, dried over $Na_2SO_4$, filtered and the solvents were removed. The residue was purified by chromatography on silica using dichloromethane/methanol (10/0.5) to afford the title compound as a pale yellow solid (0.047 g, 42 %).

$^1H$-NMR (400 MHz, DMSO-$d_6$): δ = 10.73 (s, 1H), 8.66 (s, 1H), 8.26 (s, 1H), 7.58 (d, 1H), 7.52 (d, 1H), 7.27 (s, 1H), 6.55 (d, 1H), 5.75 (t, 1H), 3.99 (d, 2H), 2.96 (t, 2H), 2.7-2.66 (m, 3H), 2.58 (m, 2H), 1.85-1.77 (m, 8H), 1.42 (s, 9H), 1.15-1.06 (m, 2H)
MS (ESI): m/z = 477.09 $(MH)^+$

**Preparative Examples 30 to 32**

Following a procedure similar to that described in Preparative Example 29, except using the bromo-derivatives and amines indicated in the table below, the following compounds were prepared.

| **Bromo-derivative** | **Amine** | **Product**<br>**Preparative Example** | **1. Yield**<br>**2. $^1H$-NMR ($CDCl_3$)**<br>**3. $MH^+$** |
|---|---|---|---|
| 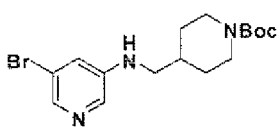 | 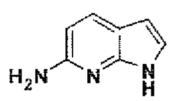 | 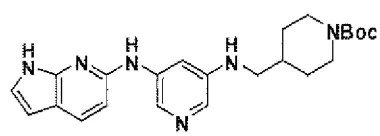<br>30 | 1. 38 %<br>2. δ = 10.27 (s, 1H), 8.09 (s, 1H), 8.26 (s, 1H), 7.73 (d, 1H), 7.58 (s, 2H), 6.88 (s, 1H), 6.61 (s, 1H), 6.31 (s, 1H), 3.94 (m, 2H), 2.82 (m, 2H), 2.57 (m, 2H), 1.58 (m, 3H), 1.0 (m, 2H)<br>3. 423.04 |

(continued)

| **Bromo-derivative** | **Amine** | **Product**<br>**Preparative Example** | **1. Yield**<br>**2. $^{1}$H-NMR ($CDCl_3$)**<br>**3. MH$^{+}$** |
|---|---|---|---|
| 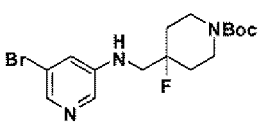 | 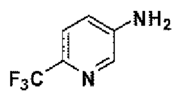 | 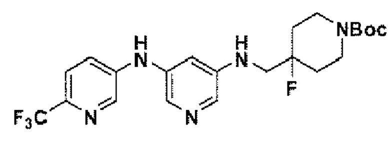<br>**31** | 1. 46 %<br>2. δ = 8.38 (d, 1H), 7.80 (dd, 2H), 7.52 (d, 1 H), 7.41 (dd, 1H), 6.74 (s, 1H), 3.96 (brs, 2H), 3.25 (dd, 2H), 3.07 (m, 2H), 1.96-1.91 (m, 2H), 1.66-1.50 (m, 2H), 1.45 (s, 9H)<br>3.470.02 |
| 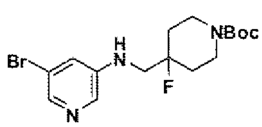 | 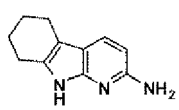 | 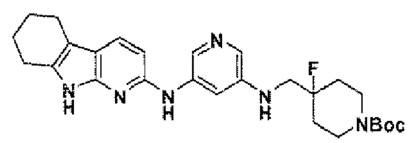<br>**32** | 1. 18 %<br>2. δ = 9.33 (s, 1H), 7.95 (s, 1H), 7.59 (d, 1H), 7.41(s, 1H), 6.81 (d, 1H), 6.58 (d, 1H), 3.93 (brs, 2H), 3.18 (d, 2H), 3.04 (m, 2H), 2.58 (m, 4H), 1.82 (m, 6H), 1.82 (m, 2H), 1.45 (s, 9H)<br>3.496.13 |

**Preparative Example 33**

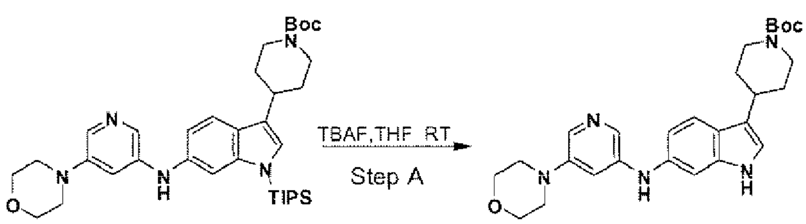


Step A

The title compound from Preparative Example 17 (0.114 g, 0.18 mmol) was dissolved in tetrahydrofuran (3 mL) and treated with a 1 M solution of tetrabutylammonium fluoride (0.198 mL, 0.198 mmol) in tetrahydrofurane. The

mixture was stirred at room temperature for 2 h and the solvents were removed. The residue was purified by chromatography on silica using a Biotage flash chromatography system (dichloromethane/methanol: 1 % -> 5%) to afford the title compound as a yellowish solid (0.067 g, 78 %).

$^1$H-NMR (400 MHz, $CDCl_3$): δ = 7.75 (s, 1H), 7.56 (s, 1H), 7.55 (d, 1H), 7.15 (s, 1H), 6.91-6.87 (m, 3H), 4.25 (brs, 2H), 3.82 (m, 4H), 3.12 (m, 4H), 2.95-2.89 (m, 3H), 2.10 (d, 2H), 1.66 (m, 2H), 1.48 (s, 9H)
MS (ESI): m/z = 478.04 $(MH)^+$

**Preparative Example 34**

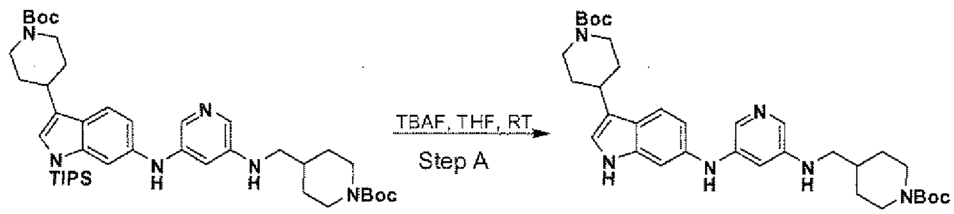


Step A

To a solution of the title compound from Preparative Example 22 (0.1 g, 0.131 mmol) in dry tetrahydrofuran (10 mL) was added a 1 M solution of tetrabutylammonium fluoride (0.197 ml, 0.197 mmol) in tetrahydrofuran. The resulting solution was stirred at room temperature for 12 h. The reaction mixture was concentrated to dryness. The residue was purified by flash chromatography using ethyl acetate to afford the title compound as a brown solid (0.061 g, 77%).

MS ESI: m/z = 605.18 $(MH)^+$

**Example 1**

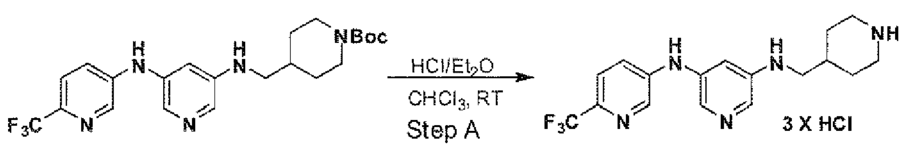


Step A

The title compound from Preparative Example 15 (0.081 g, 0.179 mmol) was dissolved in chloroform (3 mL) and treated with a 2 M solution of hydrogen chloride in diethylether (1.34 mL, 2.68 mmol, 15 equiv). The reaction mixture was stirred at room temperature overnight and the solvents were removed by syringe. The solid material was dissolved in water (5 mL) and filtered through a 0.2 μm filter cartridge. The filtrate was collected and the solvent was lyophylized to afford the title compound as a greenish glass solid (0.078 g, 94 %).

$^1$H-NMR (400 MHz, $D_2O$): δ = 8.50 (s, 1H), 7.84 (s, 2H), 7.78 (s, 1H), 7.66 (s, 1H), 7.33 (s, 1H), 3.5 (d, 2H), 3.19 (d, 2H), 3.03 (t, 2H), 2.08-2.05 (m, 3H), 1.49 (m, 2H)
MS (ESI): m/z = 351.97 $(M^+)$

**Examples 2 to 20**

Following a similar procedure as that described in Example 1, except using the compounds from the Preparative Examples indicated in the table below, the following compounds were prepared.

| Example | Compound Preparative Example | Product | 1. Yield<br>2. $^1$H-NMR ($D_2O$)<br>3. $M^+$ free base |
|---|---|---|---|
| 2 | 4 | 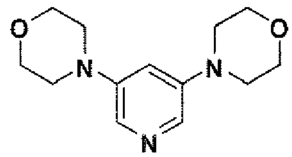 | 1. 44 %<br>2. δ = 7.79 (s, 2H), 7.33 (s, H), 3.89 (m, 8H), 3.35 (m, 8H)<br>3. 249.96 |
| 3 | 16 | 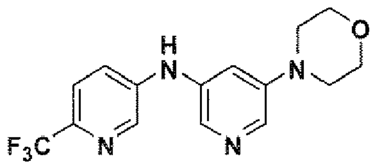 | 1. 83 %<br>2. δ = 8.46 (m, 1H), 7.95 (m, 2H), 7.81 (m, 2H), 7.56 (m, 1H), 3.91 (m, 4H), 3.39 (m, 4H) |
| 4 | 33 | 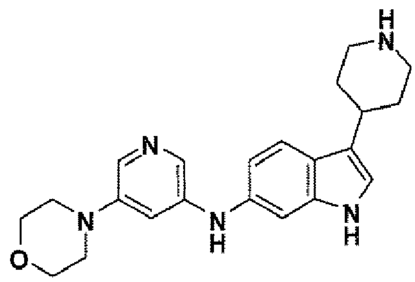 | 1. 53 %<br>2. δ = 7.72-7.63 (m, 3H), 7.31 (s, 1H), 7.25 (m, 2H), 7.01 (d, 1H), 3.83 (m, 4H), 3.59-3.54 (m, 2H), 3.21 (m, 7H), 2.31 (d, 2H), 1.95 (m, 2H) 3. 378.00 ($MH^+$) |
| 5 | 6 | 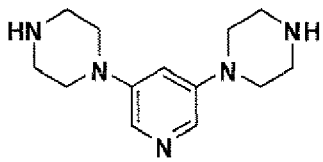 | 1. 90 %<br>2. δ = 7.86 (s, 2H), 7.42 (s, H), 3.65 (m, 8H), 3.42 (m, 8H) 3. 248.88 ($MH^+$) |
| 6 | 19 | 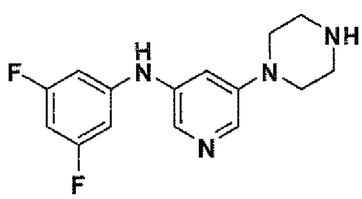 | 1. 61 %<br>2. δ = 7.92 (dd, 2H), 7.55 (s, H), 6.83 (m, 2H), 6.68 (m, 1H), 3.63 (m, 4H), 3.43 (m, 4H)<br>3. 290.92 |
| 7 | 18 | 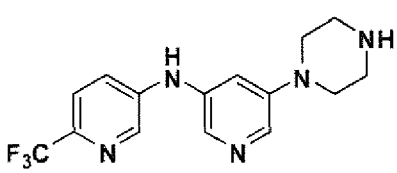 | 1. 99 %<br>2. δ = 8.49 (s, 1H), 8.00 (d, 2H), 7.83 (s, 2H), 7.64 (s, 1H), 3.66 (m, 4H), 3.44 (m, 4H)<br>3. 323.9 |

(continued)

| Example | Compound Preparative Example | Product | 1. Yield<br>2. $^1$H-NMR ($D_2O$)<br>3. $M^+$ free base |
|---|---|---|---|
| 8 | 7 | 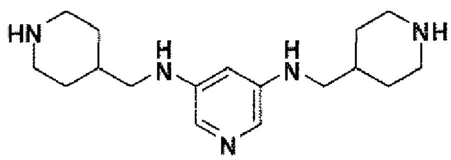 | 1. 48 %<br>2. δ = 7.96 (s, 2H), 6.49 (s, 1H), 3.19 (d, 4H), 2.86 (d, 4H), 2.72 (m, 4H), 1.76 (m, 6H), 1.19 (m, 4H) 3. 304.92 ($MH^+$) |
| 9 | 20 | 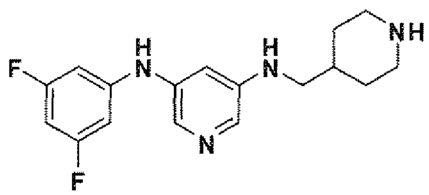 | 1. 60 %<br>2. δ = 7.66 (s, 1H), 7.55 (s, 1H), 7.22 (d, 1H), 6.82-6.77 (m, 2H), 6.67-6.65 (m, 1H), 3.47 (d, 2H), 3.14 (brs, 2H), 3.00 (m, 2H), 2.06-1.99 (m, 3H), 1.46 (m, 2H)<br>3. 318.95 |
| 10 | 21 | 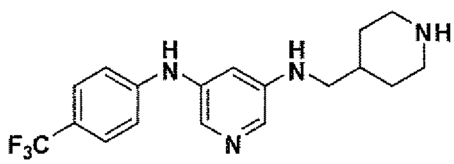 | 1. 72 %<br>2. δ = 7.71 (m, 3H), 7.55 (s, 1H), 7.34 (d, 2H), 7.25 (s, 1H), 3.48 (d, 2H), 3.14 (d, 2H), 3.00 (m, 2H), 2.06-1.99 (m, 3H), 1.46 (m, 2H)<br>3. 350.95 |
| 11 | 30 | 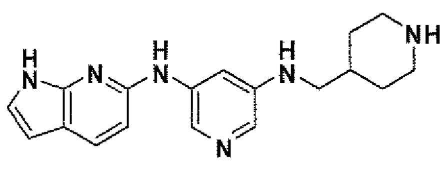 | 1. 78 %<br>2. δ = 8.28 (s, 1H), 8.06 (d, 1H), 7.46 (s, H), 7.41 (s, 1H), 7.31 (s, 1H), 6.79 (d, 1H), 6.56 (s, 1H), 3.50 (m, 2H), 3.09 (d, 2H), 3.03 (t, 2H), 2.07 (m, 2H), 1.98 (m, 1H), 1.50 (m, 2H)<br>3. 322.97 |

(continued)

| Example | Compound Preparative Example | Product | 1. Yield<br>2. $^1$H-NMR ($D_2O$)<br>3. $M^+$ free base |
|---|---|---|---|
| 12 | 34 | 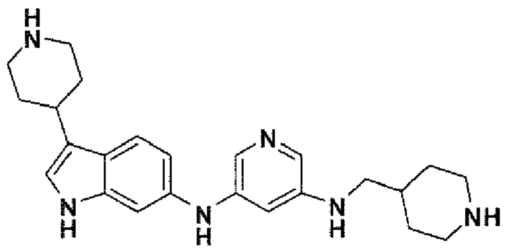 | 1. 100 %<br>2. δ = 7.62 (d, 1H), 7.39 (s, 1H), 7.28 (s, 1H), 7.24 (s, 1H), 7.14 (s, 1H), 6.92 (d, 1H), 6.91 (s, 1H), 3.45 (d, 2H), 3.33 (d, 2H), 3.12 (m, 3H), 2.97 (d, 2H), 2.86 (t, 2H), 2.21 (d, 2H), 1.75-1.92 (m, 5H), 1.25-1.42 (m, 2H)<br>3. 404.98 |
| 13 | 29 | 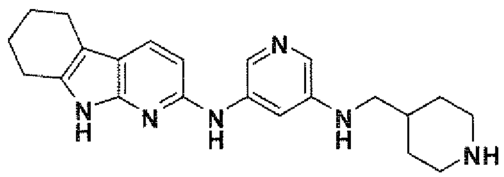 | 1. 83 %<br>2. δ = 8.02 (brs, 1H), 7.89 (d, 1H), 7.51 (d, 1H), 7.28 (s, 1H), 6.75 (d, 1H), 3.5 (d, 2H), 3.11 (d, 2H), 3.06-2.99 (m, 2H), 2.69 (m, 2H), 2.58 (m, 2H), 2.07 (d, 2H), 2.02-1.95 (m, 1H), 1.94-1.82 (m, 4H), 1.55-1.45 (m, 2H) 3. 377.04 ($MH^+$) |
| 14 | 24 | 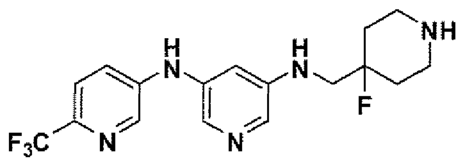 | 1. 83 %<br>2. δ = 8.47 (d, 1H), 7.80-7.79 (m, 2H), 7.76 (d, 1H), 7.70 (d, 1H), 7.42 (m, 1H), 3.56 (s, 1H), 3.51 (s, 1H), 3.45-3.40 (m, 2H), 3.29-3.22 (m, 2H), 2.29-2.23 (m, 2H), 2.02-1.84 (m, 2H)<br>3. 369.94 |
| 15 | 32 | 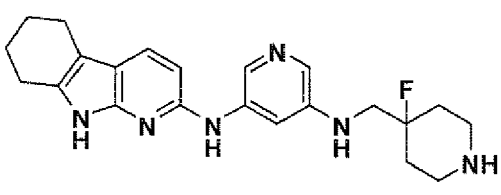 | 1. 79 %<br>2. δ = 8.12 (brs, 1H), 7.83 (d, 1H), 7.56 (s, 1H), 7.43 (s, 1H), 6.69 (d, 1H), 3.50-3.22 (m, 4H), 3.22 (m, 2H), 2.67 (m, 2H), 2.56 (m, 2H), 2.28-2.23 (m, 2H), 2.02-1.92 (m, 2H), 1.88-1.82 (m, 4H)<br>3. 395.04 |

(continued)

| Example | Compound Preparative Example | Product | 1. Yield<br>2. $^1$H-NMR ($D_2O$)<br>3. $M^+$ free base |
|---|---|---|---|
| 16 | 23 | 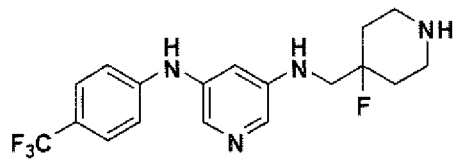 | 1. 95 %<br>2. δ = 7.64-7.62 (m, 3H), 7.57 (s, 1H), 7.27-7.23 (m, 3H), 3.51-3.42 (m, 4H), 3.28-3.22 (m, 2H), 2.27-2.22 (m, 2H), 2.02-1.88 (m, 2H)<br>3.368.97 |
| 17 | 25 | 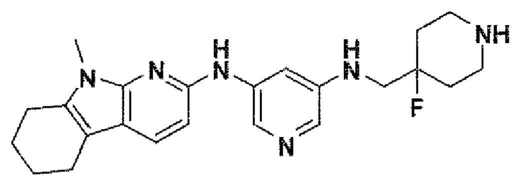 | 1. 70 %<br>2. δ = 8.04 (s, 1H), 7.58 (m, 1H), 7.44 (s, 1H), 7.34 (s, 1H), 6.48 (m,1H), 3.45 (s, 3H), 3.45 (m, 2H), 3.37 (d, 2H), 3.26 (m, 2H), 2.58 (m, 2H), 2.45 (m, 2H), 2.25 (m, 2H), 2.03-1.88 (m, 2H), 1.83 (m, 2H), 1.74 (m, 2H) 3. 409.02 $(MH)^+$ |
| 18 | 26 | 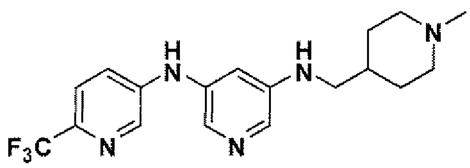 | 1. 76 %<br>2. δ = 8.48 (s, 1H), 7.81 (s, 2H), 7.76 (s, 1H), 7.63 (s, 1H), 7.31 (s, 1H), 3.55 (d, 2H), 3.16 (d, 2H), 2.99 (t, 2H), 2.85 (s, 3H), 2.08 (d, 2H), 1.96 (brs, 1H), 1.48 (q, 2H)<br>3. 365.98 |
| 19 | 27 | 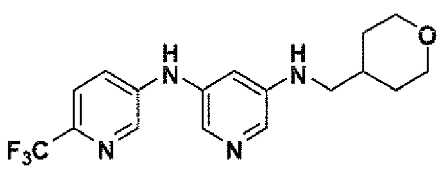 | 1. 65 %<br>2. δ = 8.33 (s, 1H), 7.74-7.68 (m, 2H), 7.65 (s, 1H), 7.55 (s, 1H), 7.16 (s, 1H), 3.97 (dd, 2H), 3.44 (t, 2H), 3.02 (d, 2H), 1.91-1.85 (m, 1H), 1.70 (d, 2H), 1.36-1.25 (m, 2H)<br>3. 352.94 |

(continued)

| Example | Compound Preparative Example | Product | 1. Yield<br>2. $^1$H-NMR ($D_2O$)<br>3. $M^+$ free base |
|---|---|---|---|
| 20 | 28 | 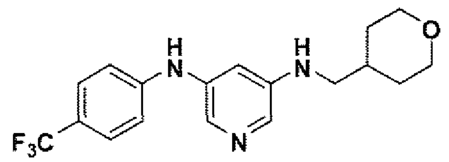 | 1. 50 %<br>2. δ = 7.56-7.52 (m, 3H), 7.42 (s, 1H), 7.16 (d, 2H), 7.04 (s, 1H), 3.97 (dd, 2H), 3.44 (t, 2H), 2.97 (d, 2H), 1.86-1.82 (m, 1H), 1.68 (d, 2H), 1.31-1.27 (m, 2H)<br>3. 351.94 |

**Determination of inhibition of Aβ1-42 aggregation**

The capacity of the compounds of the invention to inhibit the aggregation of Aβ1-42 peptide was determined by using the thioflavin T spectrofluorescence assay (ThT-assay) as described below.

*Preparation of Aβ peptide film*

Aβ1-42 lyophilized powder (Bachem) was reconstituted in hexafluoroisopropanol (HFIP) to 1 mM. The peptide solution was sonicated for 15 min at room temperature, agitated overnight, and aliquots were placed into non-siliconized microcentrifuge tubes. The HFIP was then evaporated under a stream of argon. The resulting peptide film was dried under vacuum for 10 min, tightly sealed and stored at -80˚C until used.

*Inhibition of Aβ1-42 aggregation measurement*

To assay the small molecule-mediated inhibition of Aβ1-42 aggregation the small molecules from the examples were dissolved previous to each experiment in anhydrous dimethyl sulfoxide (DMSO, Sigma-Aldrich) to reach a concentration of 7.4 mM. An Aβ1-42 peptide film was dissolved in DMSO to reach 400 μM. An assay solution in PBS was prepared in non-siliconized incubation tubes to reach the following concentrations: 330 μM small molecule, 33 μM Aβ1-42, 10 μM thioflavin T (ThT), and 12.8% DMSO. Therefore, the final molar ratio of small molecule to Aβ1-42 was 10:1. A positive control without a small molecule was prepared to measure maximum RFU. A negative control without Aβ1-42 was prepared for each small molecule. The 3-amino-pyrazole trimer (Rzepecki et. al. Synthesis 2003, 1815) was tested as a reference compound in all assays to ascertain reproducibility between independent experiments. The solutions were then incubated for 24 hrs at 37˚C, and the spectrofluorescence (relative fluorescence units; RFU) read in six replicates in black 384-well assay plates (Perkin-Elmer) on a Perkin-Elmer FluoroCount spectrofluorometer. Inhibition of aggregation is expressed as mean % inhibition ± 1 standard deviation (SD) according to the following equation:

$$\%\ \text{inhibition} = \frac{(\text{RFU of positive control} - \text{RFU of negative control}) - (\text{RFU of sample with A}\beta\text{1-42} - \text{RFU of sample without A}\beta\text{1-42}) \times 100}{(\text{RFU of positive control} - \text{RFU of negative control})}$$

The following compounds were measured:

**Table 1:** Inhibition of Aβ1-42 aggregation of preformed Aβ1-42 fibers by the compounds of the invention. The results are expressed as mean +/- standard deviation of two independent experiments.

| Example | Structure | Inhibition at 330 μM [%] |
|---|---|---|
| 1 | 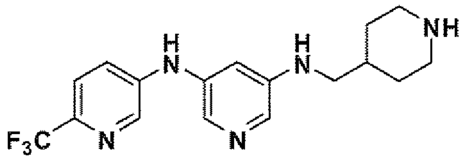 | 38.0 ± 0.6 |
| 2 | 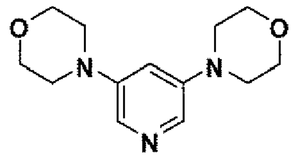 | 15.7 ± 6.1 |
| 3 | 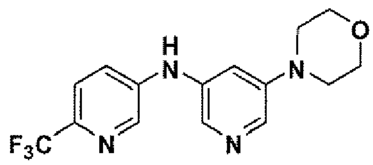 | 36.9 ± 17.2 |
| 4 | 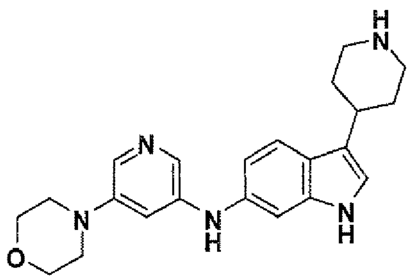 | 53.3 ± 7.6 |
| 5 | 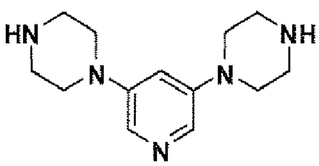 | 60.1 ± 7.8 |
| 6 | 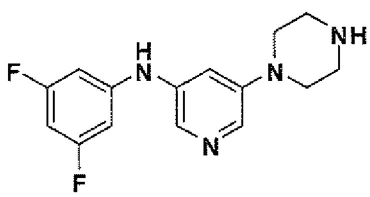 | 62.5 ± 7.4 |
| 7 | 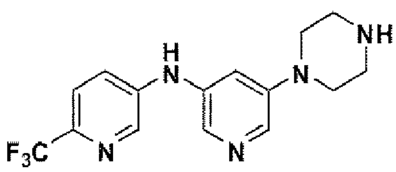 | 28.3 ± 3.2 |

(continued)

| Example | Structure | Inhibition at 330 μM [%] |
|---|---|---|
| 8 | 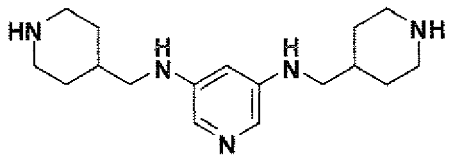 | 69.5 ± 1.4 |
| 9 | 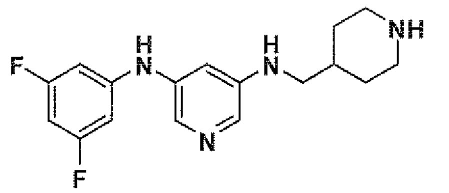 | 55.0 ± 7.4 |
| 10 | 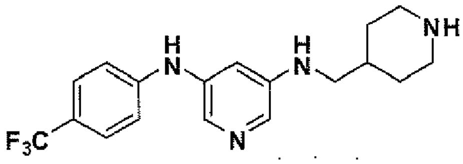 | 73.9 ± 4.7 |
| 11 | 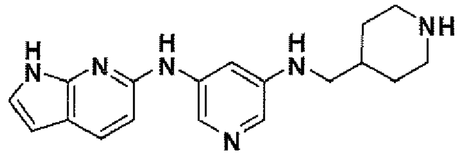 | 62.7 ± 2.1 |
| 12 | 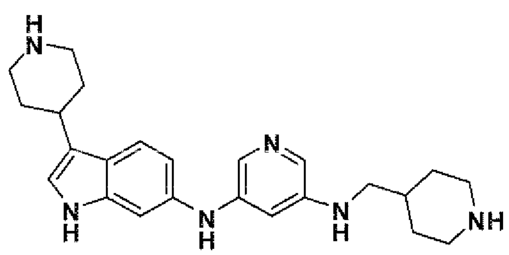 | 95.4 ± 0.8 |
| 13 | 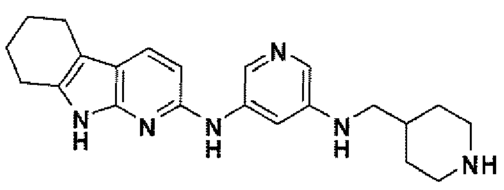 | 100.1 ± 4.4 |
| 14 | 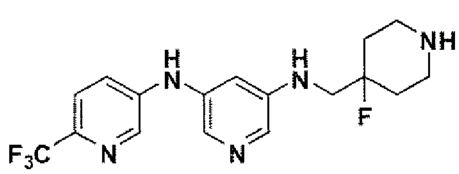 | 86.2 ± 4.7 |

(continued)

| Example | Structure | Inhibition at 330 μM [%] |
|---|---|---|
| 15 | 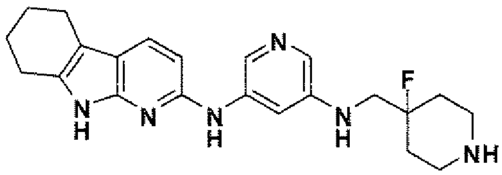 | 95.7 ± 0.4 |
| 16 | 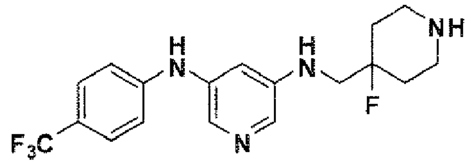 | 62.9 ± 8.2 |
| 17 | 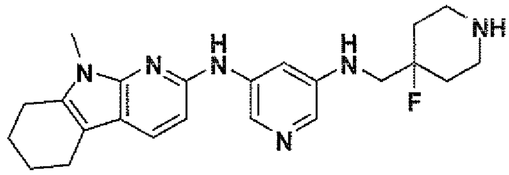 | 96.5 ± 1.4 |
| 18 | 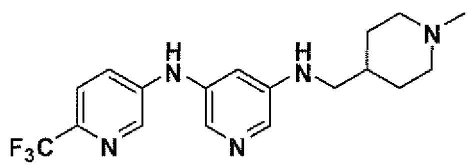 | 29.5 ± 0.1 |
| 19 | 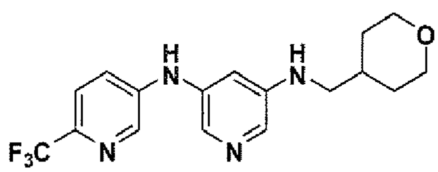 | 40.2 ± 6.3 |
| 20 | 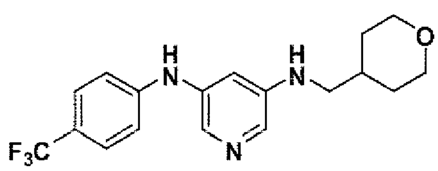 | 51.7 ± 0.3 |

## Claims

**1.** A compound of formula (I):

A-$L_1$-B (I)

and all stereoisomers, racemic mixtures, pharmaceutically acceptable salts, hydrates, solvates and polymorphs thereof;
wherein
A is selected from the group consisting of:

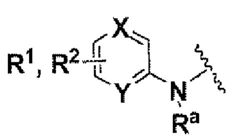
(II)
,
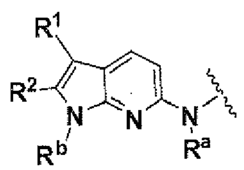
(III)
,
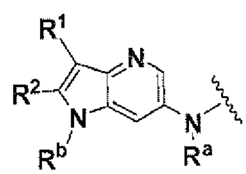
(IV)
,
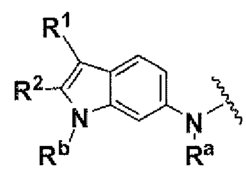
(V)

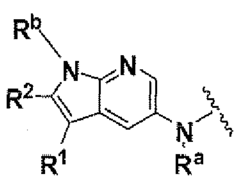
(VI)
,
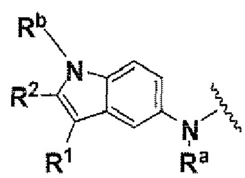
(VII)
,
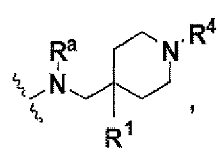
(VIII)
,
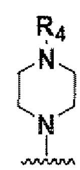
(IX)
or
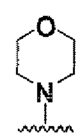
(X)

$L_1$ is:

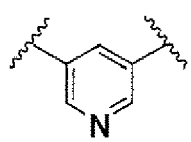
(XI)

B is selected from the group consisting of:

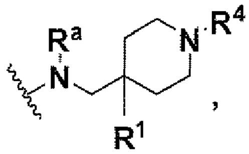, 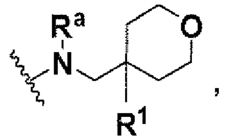, 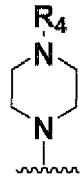 or 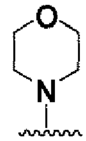

(XII) (XIII) (XIV) (XV)

wherein
for each occurrence $R^1$ and $R^2$ are each independently selected from the group consisting of hydrogen, halogen, CN, $CF_3$, $CONR^4R^5$, alkyl, cycloalkyl, cycloalkylalkyl, heterocycloalkyl, fluoroalkyl, heterocycloalkylalkyl, alkenyl, alkynyl, aryl, heteroaryl, arylalkyl and heteroarylalkyl, wherein alkyl, cycloalkyl, cycloalkylalkyl, heterocycloalkyl, fluoroalkyl, heterocycloalkylalkyl, alkenyl, alkynyl, aryl, heteroaryl, arylalkyl and heteroarylalkyl can be optionally substituted, or wherein $R^1$ and $R^2$ when taken together can form a 5- to 8-membered ring containing carbon atoms and optionally one or more heteroatoms selected from O, S, or N or optionally one or more heteroatom (e.g., N, O and/or S)-containing moieties and wherein the 5- to 8-membered ring may be substituted by $NR^{20}R^{21}$;
for each occurrence $R^3$ is each independently selected from the group consisting of: hydrogen, alkyl, cycloalkyl, cycloalkylalkyl, heterocycloalkyl, fluoroalkyl, heterocycloalkylalkyl, alkenyl, alkynyl, aryl, heteroaryl, arylalkyl, heteroarylalkyl and aminoalkyl, wherein alkyl, cycloalkyl, cycloalkylalkyl, heterocycloalkyl, fluoroalkyl, heterocycloalkylalkyl, alkenyl, alkynyl, aryl, heteroaryl, arylalkyl, heteroarylalkyl and aminoalkyl can be optionally substituted;
for each occurrence $R^a$ is independently selected from the group consisting of hydrogen, alkyl, haloalkyl, $S(O)_tNR^4R^5$, $S(O)_tR^4$, $C(O)OR^4$, $C(O)R^4$, and $C(O)NR^4R^5$;
for each occurrence $R^b$ is independently selected from the group consisting of hydrogen, alkyl, and haloalkyl;
for each occurrence $R^4$, $R^5$, $R^{20}$ and $R^{21}$ are each independently selected from the group consisting of hydrogen, alkyl, cycloalkyl, cycloalkylalkyl, heterocycloalkyl, fluoroalkyl, heterocycloalkylalkyl, alkenyl, alkynyl, aryl, heteroaryl, arylalkyl, heteroarylalkyl and aminoalkyl, wherein alkyl, cycloalkyl, cycloalkylalkyl, heterocycloalkyl, fluoroalkyl, heterocycloalkylalkyl, alkenyl, alkynyl, aryl, heteroaryl, arylalkyl, heteroarylalkyl and aminoalkyl can be optionally substituted, or wherein $R^{20}$ and $R^{21}$ when taken together with the nitrogen to which they are attached can form a 3- to 8-membered ring containing carbon atoms and optionally one or more further heteroatoms selected from O, S, or N or optionally one or more heteroatom (e.g., N, O and/or S)-containing moieties and wherein the 3- to 8-membered ring may be optionally substituted;
for each occurrence X and Y are each independently selected from the group consisting of $CR^3$ and N; and
t is 1 or 2.

**2.** The compound of claim 1, wherein $R^1$ is each independently selected from hydrogen, halogen (such as F or Cl), CN, fluoroalkyl (such as $CF_3$), and heterocycloalkyl (such as

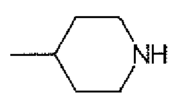 ).

**3.** The compound of claim 1, wherein $R^1$ and $R^2$ when taken together form a 6-membered ring containing carbon atoms.

**4.** The compound of claim 1 or 2, wherein $R^2$ is each independently selected from hydrogen, halogen (such as F or Cl), CN, and fluoroalkyl (such as $CF_3$).

**5.** The compound of any of the preceding claims, wherein $R^a$ is hydrogen.

**6.** The compound of any of the preceding claims, wherein $R^b$ is hydrogen or methyl.

**7.** The compound of any of the preceding claims, wherein $R^4$ is hydrogen or alkyl and/or $R^5$ is hydrogen or alkyl.

**8.** The compound of claim 1, wherein A has the formula (II), (III) or (V)

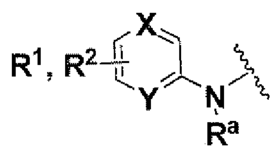

(II)

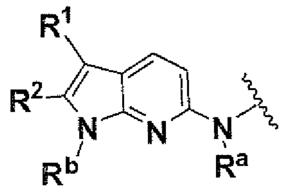

(III)

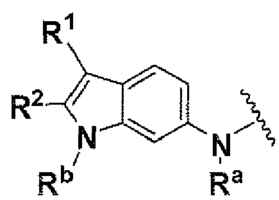

(V)

.

**9.** The compound of claim 1, wherein B has the formula (XII)

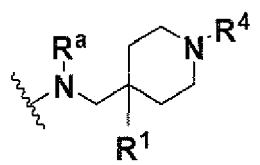

(XII)

.

**10.** The compound of claim 8, wherein A is selected from

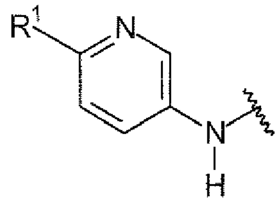

,

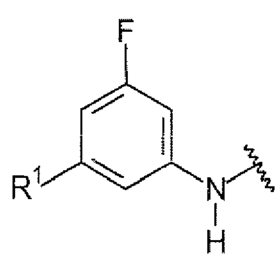

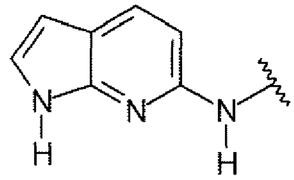

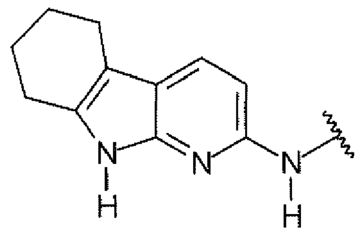

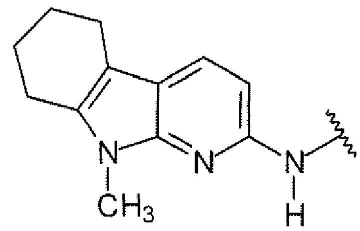

or

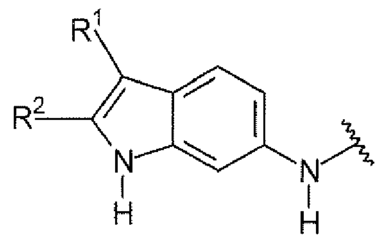

**11.** The compound of claim 9, wherein B is taken from

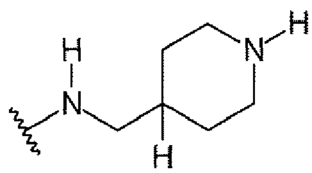

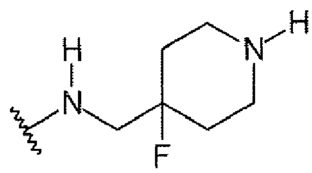

.

**12.** The compound of any of the preceding claims, wherein the compound is selected from the group consisting of

| | |
|---|---|
| 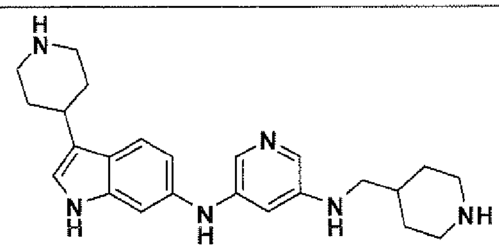 | 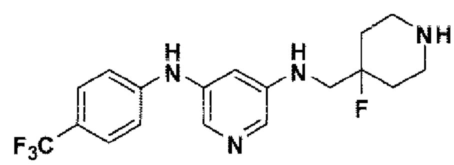 |
| 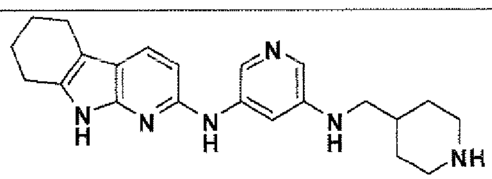 | 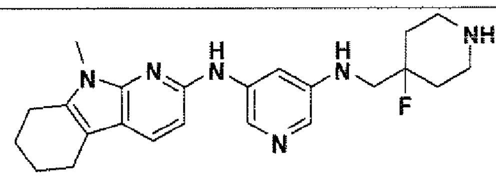 |

| | |
|---|---|
| 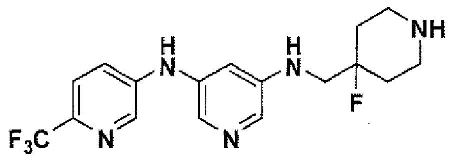 | 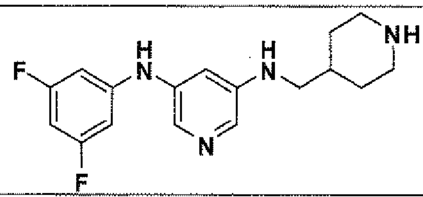 |
| 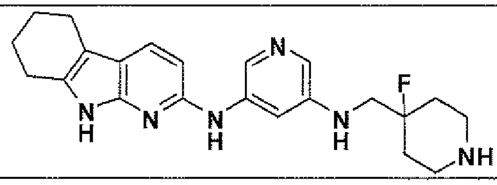 | 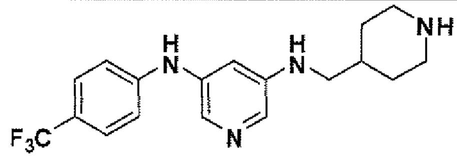 |

**13.** A compound of any one of claims 1 to 12 comprising a radionuclide.

**14.** A radiopharmaceutical formulation comprising the compound of claim 13 and optionally a pharmaceutically acceptable carrier or excipient.

**15.** A pharmaceutical composition comprising a compound of any one of claims 1 to 12 and optionally a pharmaceutically acceptable carrier or excipient.

**16.** Use of the compound of any one of claims 1 to 12 for the preparation of a medicament for treating or preventing a disease or condition associated with an amyloid and/or amyloid-like protein.

**17.** The compound of any one of claims 1 to 12 for use in treating or preventing a disease or condition associated with an amyloid and/or amyloid-like protein.

**18.** The use of claim 16 or the compound of claim 17, wherein the disease is a neurological disorder.

**19.** The use or the compound of claim 18, wherein the neurological disorder is Alzheimer's disease (AD), Lewy body dementia (LBD), Down's syndrome, hereditary cerebral hemorrhage with amyloidosis (Dutch type), the Guam Parkinson-Dementia complex or mild cognitive impairment (MCI).

**20.** The use or the compound of claim 19, wherein the neurological disorder is Alzheimer's disease.

**21.** The use of claim 16 or the compound of claim 17, wherein the disease is progressive supranuclear palsy, multiple sclerosis, inclusion-body myositis (IBM), Creutzfeld Jacob disease, Parkinson's disease, HIV-related dementia, amyotropic lateral sclerosis (ALS), inclusion-body myositis (IBM), adult onset diabetis, senile cardiac amyloidosis, endocrine tumors, glaucoma, ocular amyloidosis, primary retinal degeneration, macular degeneration (such as age-related macular degeneration (AMD)), optic nerve drusen, optic neuropathy, optic neuritis, or lattice dystrophy.

**22.** A mixture comprising a compound according to any one of claims 1 to 12 and optionally at least one further biologically active compound and/or a pharmaceutically acceptable carrier and/or a diluent and/or an excipient and wherein preferably the further biologically active compound is a compound used in the treatment of amyloidosis.

**23.** The mixture according to claim 22, wherein the further biologically active compound is selected from the group consisting of antibodies, vaccines, compounds against oxidative stress, anti-apoptotic compounds, metal chelators, inhibitors of DNA repair such as pirenzepin and metabolites, 3-amino-1-propanesulfonic acid (3APS), 1,3-propanedisulfonate (1,3PDS), α-secretase activators, β- and γ-secretase inhibitors, tau proteins, neurotransmitters, β-sheet breakers, attractants for amyloid beta clearing / depleting cellular components, inhibitors of N-terminal truncated amyloid beta including pyroglutamated amyloid beta 3-42, anti-inflammatory molecules, or cholinesterase inhibitors (ChEIs) such as tacrine, rivastigmine, donepezil, and/or galantamine, M1 agonists and other drugs including any amyloid or tau modifying drug and nutritive supplements.

**24.** The mixture according to claim 23, wherein the further biologically active compound is a cholinesterase inhibitor (ChEIs).

**25.** The mixture according to claim 23, wherein the further biologically active compound is selected from the group consisting of tacrine, rivastigmine, donepezil, galantamine, niacin and memantine.

**26.** The mixture according to claim 22, wherein the further biologically active compound is an antibody, particularly a monoclonal antibody, including any functionally equivalent antibody or functional parts thereof.

**27.** The mixture according to claim 26, wherein the antibody, particularly the monoclonal antibody, including any functionally equivalent antibody or functional parts thereof, is an antibody which binds amyloid β.

**28.** The mixture according to claim 26 or 27, wherein the antibody, particularly the monoclonal antibody, including any functionally equivalent antibody or functional parts thereof, is an antibody which antibody, upon co-incubation with amyloid monomeric and/or polymeric soluble amyloid peptides, particularly with β-amyloid monomeric peptides such as, for example, Aβ monomeric peptides 1-39; 1-40, 1-41, or 1-42, and/or a polymeric soluble β-amyloid peptide comprising a plurality of the Aβ monomeric units, but especially with an $A\beta_{1-42}$ monomeric and/or an Aβ polymeric soluble amyloid peptide comprising a plurality of the $A\beta_{1-42}$ monomeric units, inhibits the aggregation of the Aβ monomers into high molecular polymeric fibrils or filaments and, in addition, upon co-incubation with preformed high molecular polymeric amyloid fibrils or filaments formed by the aggregation of amyloid monomeric peptides, particularly β-amyloid monomeric peptides such as, for example, Aβ monomeric peptides 1-39; 1-40, 1-41, or 1-42, but especially $A\beta_{1-42}$ monomeric peptides, is capable of disaggregating preformed polymeric fibrils or filaments.

**29.** The mixture according to claim 26, wherein the antibody is a chimeric antibody or a functional part thereof, or a humanized antibody or a functional part thereof.

**30.** The mixture according to claim 26, wherein the antibody is a monoclonal antibody selected from the group of antibodies having the characteristic properties of an antibody produced by the hybridoma cell line:

a) FP 12H3, deposited on December 01, 2005 and December 09, 2005, respectively, as DSM ACC2752;
b) FP 12H3-C2, deposited on December 01, 2005 and December 09, 2005, respectively, as DSM ACC2750;
c) FP 12H3-G2, deposited on December 01, 2005 and December 09, 2005, respectively, as DSM ACC2751;
d) ET 7E3, deposited on December 08, 2005 as DSM ACC2755; and
e) EJ 7H3, deposited on December 08, 2005 as DSM ACC2756

or wherein the antibody is a monoclonal antibody selected from the group of antibodies produced by the hybridoma cell line:

a) FP 12H3, deposited on December 01, 2005 and December 09, 2005, respectively, as DSM ACC2752;
b) FP 12H3-C2, deposited on December 01, 2005 and December 09, 2005, respectively, as DSM ACC2750;
c) FP 12H3-G2, deposited on December 01, 2005 and December 09, 2005, respectively, as DSM ACC2751;
d) ET 7E3, deposited on December 08, 2005 as DSM ACC2755; and
e) EJ 7H3, deposited on December 08, 2005 as DSM ACC2756.

**31.** The mixture according to claim 26, wherein the antibody is a humanized antibody exhibiting a light chain and a heavy chain as depicted in SEQ ID No. 13 and SEQ ID No. 16 of International Application No. PCT/US2007/073504 or wherein the antibody is a humanized antibody exhibiting a light chain variable region and a heavy chain variable region as depicted in SEQ ID No. 12 and SEQ ID No. 15 of International Application NO. PCT/US2007/073504.

**32.** The mixture according to claim 22, wherein the further biologically active compound is an Aβ antigenic peptide fragment consisting of a single or repetitive stretch of a plurality of contiguous amino acid residues from the N-terminal part of the Aβ peptide, particularly a stretch of between 13 and 15 contiguous amino acid residues, and preferably wherein the Aβ antigenic peptide fragment is an $A\beta_{1-15}$ peptide antigen.

**33.** The mixture according to claim 23, wherein the $A\beta_{1-15}$ peptide antigen is a palmitoylated $A\beta_{1-15}$ peptide antigen modified by covalently attached palmitoyl residues, particularly between 2 and 4, more particularly 4 residues, at each end of the peptide reconstituted in a liposome.

**34.** The mixture according to any one of claims 22 to 33, wherein the compound and/or the further biologically active compound are present in a therapeutically effective amount.

**35.** A method of collecting data for the diagnosis of an amyloid-associated disease or condition in a sample or a patient comprising:

(a) bringing a sample or a specific body part or body area suspected to contain an amyloid protein into contact with a compound according to any one of claims 1 to 12;
(b) allowing the compound to bind to the amyloid protein;
(c) detecting the compound bound to the protein; and
(d) optionally correlating the presence or absence of compound binding with the amyloid protein with the presence or absence of amyloid protein in the sample or specific body part or body area.

**36.** A method of determining the extent of amyloidogenic plaque burden in a tissue and/or a body fluid comprising:

(a) providing a sample representative of the tissue and/or body fluid under investigation;
(b) testing the sample for the presence of amyloid protein with a compound according to any one of claims 1 to 12;
(c) determining the amount of compound bound to the amyloid protein; and
(d) calculating the plaque burden in the tissue and/or body fluid.

**37.** The method according to claim 36, wherein the determination in step (c) is conducted such that presence or absence of the compound binding with the amyloid protein correlates with presence or absence of amyloid protein.

**38.** A method of collecting data for determining a predisposition to an amyloid-associated disease or condition in a patient comprising detecting the specific binding of a compound according to any one of claims 1 to 12 to an amyloid protein in a sample or *in situ* which comprises the steps of:

(a) bringing the sample or a specific body part or body area suspected to contain the amyloid protein into contact with a compound according to any one of claims 1 to 12, which compound specifically binds to the amyloid protein;
(b) allowing the compound to bind to the amyloid protein to form a compound/protein complex;
(c) detecting the formation of the compound/protein complex;
(d) optionally correlating the presence or absence of the compound/protein complex with the presence or absence of amyloid protein in the sample or specific body part or body area; and
(e) optionally comparing the amount of the compound/protein complex to a normal control value.

**39.** A method of collecting data for monitoring minimal residual disease in a patient following treatment with an antibody or a vaccine composition, wherein the method comprises:

(a) bringing a sample or a specific body part or body area suspected to contain an amyloid protein into contact with a compound according to any one of claims 1 to 12, which compound specifically binds to the amyloid protein;
(b) allowing the compound to bind to the amyloid protein to form a compound/protein complex;
(c) detecting the formation of the compound/protein complex;
(d) optionally correlating the presence or absence of the compound/protein complex with the presence or absence of amyloid protein in the sample or specific body part or body area; and
(e) optionally comparing the amount of the compound/protein complex to a normal control value.

**40.** A method of collecting data for predicting responsiveness of a patient being treated with an antibody or a vaccine composition comprising:

(a) bringing a sample or a specific body part or body area suspected to contain an amyloid protein into contact with a compound according to any one of claims 1 to 12, which compound specifically binds to the amyloid protein;
(b) allowing the compound to bind to the amyloid protein to form a compound/protein complex;
(c) detecting the formation of the compound/protein complex;
(d) optionally correlating the presence or absence of the compound/protein complex with the presence or absence of amyloid protein in the sample or specific body part or body area; and
(e) optionally comparing the amount of the compound/protein complex to a normal control value.

**41.** A test kit for detection and/or diagnosis of an amyloid-associated disease or condition comprising a compound according to any one of claims 1 to 12.

**42.** The test kit according to claims 41 comprising a container holding one or more compounds according to any one

of claims 1 to 12 and instructions for using the compound for the purpose of binding to an amyloid protein to form a compound/protein complex and detecting the formation of the compound/protein complex such that presence or absence of the compound/protein complex correlates with the presence or absence of the amyloid protein.

**43.** Use of the compound of any one of claims 1 to 12 for the preparation of a medicament for treating or preventing an ocular disease or condition associated with a pathological abnormality/change in the tissue of the visual system, particularly associated with an amyloid-beta-related pathological abnormality/change in the tissue of the visual system.

**44.** The use of claim 43, wherein the ocular disease or condition is selected from the group consisting of neuronal degradation, cortical visual deficits, glaucoma, cataract due to beta-amyloid deposition, ocular amyloidosis, primary retinal degeneration, macular degeneration, for example age-related macular degeneration, optic nerve drusen, optic neuropathy, optic neuritis, and lattice dystrophy.

**45.** A compound of any of claims 1 to 12 for use in treating or preventing an ocular disease or condition associated with a pathological abnormality/change in the tissue of the visual system, particularly associated with an amyloid-beta-related pathological abnormality/change in the tissue of the visual system.

**46.** The compound of claim 45, wherein the ocular disease or condition is selected from the group consisting of neuronal degradation, cortical visual deficits, glaucoma, cataract due to beta-amyloid deposition, ocular amyloidosis, primary retinal degeneration, macular degeneration, for example age-related macular degeneration, optic nerve drusen, optic neuropathy, optic neuritis, and lattice dystrophy.

**47.** A compound of any of claims 1 to 12 for use in inhibiting protein aggregation, in particular for use in inhibiting Aβ1-42 aggregation, Tau aggregation or alpha-synuclein aggregation.

Europäisches Patentamt
European Patent Office
Office européen des brevets

# EUROPEAN SEARCH REPORT

Application Number
EP 11 16 2677

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 99/21834 A1 (NEUROSEARCH AS [DK]; PETERS DAN [SE]; OLSEN GUNNAR M [DK]; NIELSEN SIM) 6 May 1999 (1999-05-06)<br>* compound 4A page 31 *<br>* claims 4,11 *<br>----- | 1-8, 15-47 | INV.<br>C07D401/04<br>C07D401/12<br>C07D401/14<br>C07D413/14<br>C07D471/04<br>A61K31/496<br>A61P25/00 |
| X | DESMARETS C ET AL: "Nickel-catalysed synthesis of 3-chloroanilines and chloro aminopyridines via cross-coupling reactions of aryl and heteroaryl dichlorides with amines",<br>TETRAHEDRON LETTERS, ELSEVIER, AMSTERDAM, NL,<br>vol. 42, no. 2,<br>8 January 2001 (2001-01-08), pages 247-250, XP004227747,<br>ISSN: 0040-4039, DOI: 10.1016/S0040-4039(00)01931-6<br>* compounds 3b, 4b, *<br>* Table 2 entry 8 *<br>----- | 1-7 | |
| | | | TECHNICAL FIELDS SEARCHED (IPC) |
| A | WO 2009/103652 A1 (HOFFMANN LA ROCHE [CH]; BAUMANN KARLHEINZ [DE]; FLOHR ALEXANDER [DE];) 27 August 2009 (2009-08-27)<br>* the whole document *<br>----- | 1-47 | C07D<br>A61K<br>A61P |

1

The present search report has been drawn up for all claims

| Place of search | Date of completion of the search | Examiner |
|---|---|---|
| The Hague | 6 September 2011 | Diederen, Jeroen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 16 2677

This annex lists the patent family members relating to the patent documents cited in the above-mentioned European search report.
The members are as contained in the European Patent Office EDP file on
The European Patent Office is in no way liable for these particulars which are merely given for the purpose of information.

06-09-2011

| Patent document cited in search report | Publication date | Patent family member(s) | Publication date |
|---|---|---|---|
| WO 9921834 A1 | 06-05-1999 | AT 491689 T | 15-01-2011 |
| | | AT 278670 T | 15-10-2004 |
| | | AU 744539 B2 | 28-02-2002 |
| | | BR 9813279 A | 22-08-2000 |
| | | CA 2306093 A1 | 06-05-1999 |
| | | CN 1277604 A | 20-12-2000 |
| | | CZ 20001507 A3 | 16-08-2000 |
| | | DE 69826883 D1 | 11-11-2004 |
| | | DE 69826883 T2 | 03-02-2005 |
| | | DK 1027336 T3 | 24-01-2005 |
| | | EE 200000178 A | 16-04-2001 |
| | | EP 1491532 A1 | 29-12-2004 |
| | | EP 1027336 A1 | 16-08-2000 |
| | | ES 2230723 T3 | 01-05-2005 |
| | | HK 1031378 A1 | 23-04-2004 |
| | | HU 0100426 A2 | 28-02-2002 |
| | | IL 135108 A | 31-12-2006 |
| | | IS 5420 A | 28-03-2000 |
| | | JP 4570773 B2 | 27-10-2010 |
| | | JP 2001521025 A | 06-11-2001 |
| | | NO 20002132 A | 26-04-2000 |
| | | NZ 503520 A | 28-08-2002 |
| | | PL 340224 A1 | 15-01-2001 |
| | | PT 1027336 E | 31-01-2005 |
| | | RU 2205179 C2 | 27-05-2003 |
| | | SK 6142000 A3 | 12-09-2000 |
| | | TR 200001171 T2 | 23-10-2000 |
| | | US 6825189 B1 | 30-11-2004 |
| WO 2009103652 A1 | 27-08-2009 | AR 070437 A1 | 07-04-2010 |
| | | AU 2009216851 A1 | 27-08-2009 |
| | | CA 2713716 A1 | 27-08-2009 |
| | | CN 101952275 A | 19-01-2011 |
| | | EP 2257541 A1 | 08-12-2010 |
| | | JP 2011512380 A | 21-04-2011 |
| | | KR 20100118118 A | 04-11-2010 |
| | | PE 14722009 A1 | 24-09-2009 |
| | | US 2009215759 A1 | 27-08-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004058258 A
- WO 2007068412 A
- US 2007073504 W
- WO 2007068411 A
- WO 9613590 A, Maertens and Stuyver
- WO 9629605 A, Zrein


### Non-patent literature cited in the description


- *Experimental Eye Research,* 2004, vol. 78, 243-256
- Remington's Pharmaceutical Sciences. Mack Publishing Co, 1991
- **RZEPECKI et al.** *J. Biol. Chem.,* 2004, vol. 279 (46), 47497-47505
- **HARLOW ; LANE.** Antibodies: A Laboratory Manual. Cold Spring Harbor Laboratory, 555-612
- **KENNEDY, J. H. et al.** *Clin. Chim. Acta,* 1976, vol. 70, 1-31
- **SCHURS, A. H. W. M. et al.** *Clin. Chim Acta,* 1977, vol. 81, 1-40
- **DING, J. et al.** Targeting age-related macular degeneration with Alzheimer's disease based immunotherapies: Anti-amyloid-b antibody attenuates pathologies in an age-related macular degeneration mouse model. *Vision Research,* 2007
- **RZEPECKI.** *Synthesis,* 2003, 1815